# EUROPEAN PATENT APPLICATION

(11) **EP 4 286 851 A1**
(43) Date of publication of application: **06.12.2023**
(21) Application number: 22176741.1
(22) Date of filing: 01.06.2022
(51) Int. Cl.: G01N 33/569, C12Q 1/6883

(54) **METHODS AND MEANS FOR MOLECULAR CHARACTERIZATION OF POST-VIRAL FATIGUE SYNDROME**

(71) Applicant: Universität Wien, 1010 Wien (AT); Medizinische Universität Wien, 1090 Wien (AT)
(72) Inventor: GERNER, Christopher, 1090 Wien (AT); SCHMETTERER, Klaus, 1090 Wien (AT); MEIER-MENCHES, Samuel, 1090 Wien (AT); KOVARIK, Johannes, 1090 Wien (AT); BILECK, Andrea, 1090 Wien (AT); PAVONE-GYÖNGYÖSI, Mariann, 1090 Wien (AT)
(74) Representative: Loidl, Manuela Bettina

(57) **Abstract**

The invention refers to molecular characterization of blood samples for predicting or diagnosing post-viral fatigue syndrome. The invention further refers to a method for monitoring treatment of post-viral fatigue syndrome and a method for identifying a patient eligible for treatment of post-viral fatigue syndrome. The invention further refers to a post-viral fatigue syndrome specific multimarker panel and a diagnostic kit comprising the multimarker panel.

## Description

### FIELD OF THE INVENTION

The invention refers to molecular characterization of blood samples for predicting or diagnosing post-viral fatigue syndrome. The invention further refers to a method for monitoring treatment of post-viral fatigue syndrome and a method for identifying a patient eligible for treatment of post-viral fatigue syndrome. The invention further refers to a post-viral fatigue syndrome specific multimarker panel and a diagnostic kit comprising the multimarker panel.

### BACKGROUND OF THE INVENTION

While the potential impact of post-viral fatigue syndrome such as chronic post-COVID-19 syndrome or long-COVID for our society becomes increasingly evident, no disease mechanism nor any kind of diagnosis based on molecular parameters has been established up until now.

The outbreak of the COVID-19 pandemic in late 2019 has led to an unprecedented worldwide health crisis with rapid spread of a novel pathogenic member of the coronavirus family, termed SARS-CoV-2, infecting more than 450 million people worldwide (www.who.int/emergencies/diseases/). Acute SARS-CoV-2 infection may induce an inappropriate and unique inflammatory response causing the pathognomonic severe respiratory symptoms which can be further accompanied by damage of multiple organs such as brain, heart and kidneys. Accordingly, acute COVID-19 infection may cause high lethality claiming more than 6 million deaths worldwide so far (www.who.int/emergencies/diseases/). Rather disturbingly, it has also become evident, that not all patients fully recover following SARS-CoV-2 infection. It has already been recognized that chronic persistence of COVID-19 symptoms after acute infection may constitute a novel somatic disease entity, typically termed long-COVID syndrome (LCS) although no exact definition exists. Typically, LCS patients display general fatigue, lack of concentration and physical fitness, dyspnea as well as a broad range of other clinical symptoms throughout the whole organism, which severely impedes the quality of life of affected patients. These clinical presentations mirror the situations found in chronic fatigue syndrome which can be secondary to infection with different viruses and was also found in other past coronavirus epidemics including Middle East Respiratory Syndrome (MERS) and Severe Acute Respiratory Syndrome (SARS). Especially for the latter symptom persistence for up to years has been observed. Strikingly, development of LCS is not associated with disease severity and so far neither distinct risk factors nor causative comorbidities or a causal therapy have been identified. To date, the pathophysiology of this disease remains rather elusive. The limited set of available studies on LCS have indicated chronic tissue damage accompanied by various signs of chronic inflammation following recovery from acute COVID-19 infection. Yet, most of these studies remain rather associative and no general consent about the management of LCS has emerged so far. This may also be owed to the formal lack of definition of the disease, the rather heterogeneous presentation of symptoms and the lack of awareness for this condition, which allows the speculation that most cases so far remain unrecognized and therefore unstudied. In the light of the high infection rate worldwide, it can be expected that the prevalence of LCS will massively increase leading to a further COVID-19 associated long-term challenge and burden for the healthcare system.

Overmyer et al. (2020) describe a large-scale multi-omics analysis of COVID-19 severity. Thereby, blood samples from COVID-19 positive and negative patients with diverse disease severities have been analyzed. Overmyer et al. (2020) found a decrease of abundance of SERPINA5 with disease severity. However, this protein was listed as regulated amongst a large number of other proteins and not related to long-COVID or fatigue syndrome.

Vijayakumar et al. (2022) describe that immuno-proteomic profiling reveals aberrant immune cell regulation in the airways of individuals with ongoing post-COVID-19 respiratory disease. Thereby, BAL and plasma normalized protein abundance (NPX) expression for the 5 most significantly differentially abundance proteins between post-COVID-19 patients and healthy controls were described showing difference in the abundance of SERPINA5 between COVID-19 patients and healthy controls in BAL. However, no significant difference is shown for plasma samples for the abundance of SERPINA5 between COVID-19 patients and healthy controls.

Due to a lack of understanding of the responsible pathomechanism of long-COVID, no standard diagnosis of long-COVID has been established so far.

Thus, there is an unmet need in the art for defined biomarkers for the diagnosis of post-viral fatigue syndrome such as LCS in a human subject as well as for the monitoring of treatment of patients suffering from post-viral fatigue syndrome and for well-founded therapy decisions.

### SUMMARY OF THE INVENTION

It is the object of the invention to provide a fast, reliable *in vitro* method of determining post-viral fatigue syndrome in a human subject. A specific objective is to provide a reliable diagnostic test for prediction or diagnosis of post-viral fatigue syndrome, in particular for the purpose of specific therapy guidance, stratification, monitoring and/or control in a patient suffering from post-viral fatigue syndrome and receiving treatment with one or more therapeutics.

The objective is solved by the subject of the claims and as further described herein.

The inventors of the present invention surprisingly found a distinct multi-omics signature demonstrating a prevalence of anti-inflammatory effector molecules in plasma of long-COVID patients, offering a unique chance for diagnosing such disease state with selected molecular biomarkers and aiding the development of urgently required treatment options. Thereby, the inventors surprisingly found that long-COVID patients suffer from a predominant anti-inflammatory state rather than exhausting inflammation. In addition, monitoring of these selected biomarkers in blood sample of patients will guide the development of causal therapeutic interventions for the treatment of long-COVID.

According to the invention there is provided a method for predicting post-viral fatigue syndrome in a human subject comprising determining the production of serine protease inhibitor SERPINA5 in a blood sample of said subject, wherein a reduced production is indicative of post-viral fatigue syndrome.

Specifically, in the method for predicting post-viral fatigue syndrome, the post-viral fatigue syndrome is chronic post-COVID-19 syndrome or long-COVID.

Specifically, in the method for predicting post-viral fatigue syndrome, the reduced production of SERPINA5 is indicative of post-viral fatigue syndrome if the production of SERPINA5 is reduced by 30 % compared to a predetermined reference value.

Specifically, in the method for predicting post-viral fatigue syndrome, the blood sample is whole blood, serum, plasma, or a fraction of any of the foregoing.

Specifically, the method for predicting post-viral fatigue syndrome is further comprising determining the production of at least one marker selected from tryptophan-betaine, taurine, valine, biotinidase (BTD), and vitamin D binding protein, preferably wherein:
a. increased production of tryptophan-betaine;
b. increased production of taurine;
c. reduced production of valine;
d. reduced production of biotinidase; and/or
e. increased production of vitamin D binding protein
are indicative of post-viral fatigue syndrome.

Specifically, in the method for predicting post-viral fatigue syndrome, production of SERPINA5, tryptophan-betaine, taurine, valine, biotinidase, and vitamin D binding protein is determined, and
a. reduced production of SERPINA5;
b. increased production of tryptophan-betaine;
c. increased production of taurine;
d. reduced production of valine;
e. reduced production of biotinidase; and
f. increased production of vitamin D binding protein
are indicative of post-viral fatigue syndrome.

Specifically, in the method for predicting post-viral fatigue syndrome, production is determined by measuring protein production, RNA production, or metabolite production, preferably quantitatively or semi-quantitatively, more preferably by immunoassays, targeted or untargeted assays based on mass spectrometry hyphenated with chromatography.

Specifically, in the method for predicting post-viral fatigue syndrome, the subject receives a standard treatment of post-viral fatigue syndrome, chronic fatigue syndrome, chronicpost-COVID-19 syndrome, or long-COVID.

Specifically, in the method for predicting post-viral fatigue syndrome, the subject is monitored by determining and comparing the production of the marker(s) at different time points, preferably wherein the blood samples are drawn at least twice, at two or more different time points.

According to the invention there is further provided a method for diagnosing post-viral fatigue syndrome in a human subject comprising determining the production of SERPINA5 in a blood sample of said subject, wherein a reduced production of SERPINA5 is indicative of post-viral fatigue syndrome.

Specifically, in the method for diagnosing post-viral fatigue syndrome, the post-viral fatigue syndrome is chronic post-COVID-19 syndrome or long-COVID.

Specifically, in the method for diagnosing post-viral fatigue syndrome, the reduced production of SERPINA5 is indicative of post-viral fatigue syndrome if the production of SERPINA5 is reduced by 30 % compared to a predetermined reference value.

Specifically, in the method for diagnosing post-viral fatigue syndrome, the blood sample is whole blood, serum, plasma, or a fraction of any of the foregoing.

Specifically, in the method for diagnosing post-viral fatigue syndrome, the production of at least one further marker selected from tryptophan-betaine, taurine, valine, biotinidase BTD, and vitamin D binding protein is determined, preferably wherein
i. increased production of tryptophan-betaine;
ii. increased production of taurine;
iii. reduced production of valine;
iv. reduced production of biotinidase; and/or
v. increased production of vitamin D binding protein
are indicative of post-viral fatigue syndrome.

Specifically, in the method for diagnosing post-viral fatigue syndrome, the production of SERPINA5, tryptophan-betaine, taurine, valine, biotinidase, and vitamin D binding protein is determined, and
i. reduced production of SERPINA5;
ii. increased production of tryptophan-betaine;
iii. increased production of taurine;
iv. reduced production of valine;
v. reduced production of biotinidase; and
vi. increased production of vitamin D binding protein
are indicative of post-viral fatigue syndrome.

Specifically, in the method for diagnosing post-viral fatigue syndrome, the production is determined by measuring protein production, produced RNA, or produced metabolite, preferably quantitatively or semi-quantitatively, more preferably by immunoassays, targeted or untargeted assays based on mass spectrometry hyphenated with chromatography.

Specifically, in the method for diagnosing post-viral fatigue syndrome, the subject receives a standard treatment of post-viral fatigue syndrome, chronic fatigue syndrome, chronic post-COVID-19 syndrome, or long-COVID.

Specifically, in the method for diagnosing post-viral fatigue syndrome, the subject is monitored by determining and comparing the production of the marker(s) at different time points, preferably wherein the blood samples are drawn at least twice, at two or more different time points.

According to the invention there is further provided a method for monitoring treatment of a post-viral fatigue syndrome in a patient comprising determining the production of SERPINA5 in a blood sample of said patient, wherein increase of the production of SERPINA5 is indicative of the patient's response to said treatment.

Specifically, in the method for monitoring treatment of a post-viral fatigue syndrome, the post-viral fatigue syndrome is chronic post-COVID-19 syndrome or long-COVID.

Specifically, in the method for monitoring treatment of a post-viral fatigue syndrome, the increased production of SERPINA5 is indicative of the patient's response to said treatment if the production of SERPINA5 is increased by 20 % compared to a reference sample.

Specifically, in the method for monitoring treatment of a post-viral fatigue syndrome, the blood sample is whole blood, serum, plasma, or a fraction of any of the foregoing.

Specifically, in the method for monitoring treatment of a post-viral fatigue syndrome, the production of at least one further marker selected from tryptophan-betaine, taurine, valine, biotinidase, and vitamin D binding protein is determined, preferably wherein
i. reduced production of tryptophan-betaine;
ii. reduced production of taurine;
iii. increased production of valine;
iv. increased production of biotinidase; and/or
v. reduced production of vitamin D binding protein
are indicative of the patient's response to said treatment.

Specifically, in the method for monitoring treatment of a post-viral fatigue syndrome, the production of SERPINA5, tryptophan-betaine, taurine, valine, biotinidase, and vitamin D binding protein is determined, and
i. increased production of SERPINA5;
ii. reduced production of tryptophan-betaine;
iii. reduced production of taurine;
iv. increased production of valine;
v. increased production of biotinidase; and
vi. reduced production of vitamin D binding protein
are indicative of the patient's response to said treatment.

Specifically, in the method for monitoring treatment of a post-viral fatigue syndrome, the expression is determined by measuring protein production, RNA production, or metabolite production, preferably quantitatively or semi-quantitatively, more preferably by immunoassays, targeted or untargeted assays based on mass spectrometry hyphenated with chromatography.

Specifically, in the method for monitoring treatment of a post-viral fatigue syndrome, the patient receives a standard treatment of post-viral fatigue syndrome, chronic fatigue syndrome, chronicpost-COVID-19 syndrome, or long-COVID.

Specifically, in the method for monitoring treatment of a post-viral fatigue syndrome, the patient is monitored by determining and comparing the production of the marker(s) at different time points, preferably wherein the blood samples are drawn at least twice, at two or more different time points.

According to the invention there is further provided a method for identifying a patient eligible for treatment of post-viral fatigue syndrome by determining the production of SERPINA5 in a blood sample of said patient, wherein a reduced production of SERPINA5 is identifying said patient being eligible for treatment.

Specifically, in method for identifying a patient eligible for treatment, the post-viral fatigue syndrome is chronic post-COVID-19 syndrome or long-COVID.

Specifically, in method for identifying a patient eligible for treatment, the reduced production of SERPINA5 is indicative of post-viral fatigue syndrome if the production of SERPINA5 is reduced by 30 % compared to a predetermined reference value.

Specifically, in method for identifying a patient eligible for treatment, the blood sample is whole blood, serum, plasma, or a fraction of any of the foregoing.

Specifically, in method for identifying a patient eligible for treatment, the production of at least one further marker selected from tryptophan-betaine, taurine, valine, biotinidase, and vitamin D binding protein is determined, preferably wherein
i. increased production of tryptophan-betaine;
ii. increased production of taurine;
iii. reduced production of valine;
iv. reduced production of biotinidase; and/or
v. increased production of vitamin D binding protein
are identifying said patient being eligible for treatment.

Specifically, in method for identifying a patient eligible for treatment, the production of SERPINA5, tryptophan-betaine, taurine, valine, biotinidase BTD, and vitamin D binding protein is determined, and
i. reduced production of SERPINA5;
ii. increased production of tryptophan-betaine;
iii. increased production of taurine;
iv. reduced production of valine;
v. reduced production of biotinidase; and
vi. increased production of vitamin D binding protein
are identifying said patient being eligible for treatment.

Specifically, in method for identifying a patient eligible for treatment, the production is determined by measuring protein production, RNA production, or metabolite production, preferably quantitatively or semi-quantitatively, more preferably by immunoassays, targeted or untargeted assays based on mass spectrometry hyphenated with chromatography.

Specifically, in method for identifying a patient eligible for treatment, the patient receives a standard treatment of post-viral fatigue syndrome, chronic fatigue syndrome, chronic post-COVID-19 syndrome, or long-COVID.

Specifically, in method for identifying a patient eligible for treatment, the patient is monitored by determining and comparing the production of the marker(s) at different time points, preferably wherein the blood samples are drawn at least twice, at two or more different time points.

According to the invention there is further provided a post-viral fatigue syndrome specific multimarker panel comprising SERPINA5, and at least one of tryptophan-betaine, taurine, valine, biotinidase, and/or vitamin D binding protein.

According to the invention there is further provided a diagnostic kit comprising the multimarker panel of the invention and means as necessary for determining the production of each marker of said multimarker panel.

### FIGURES

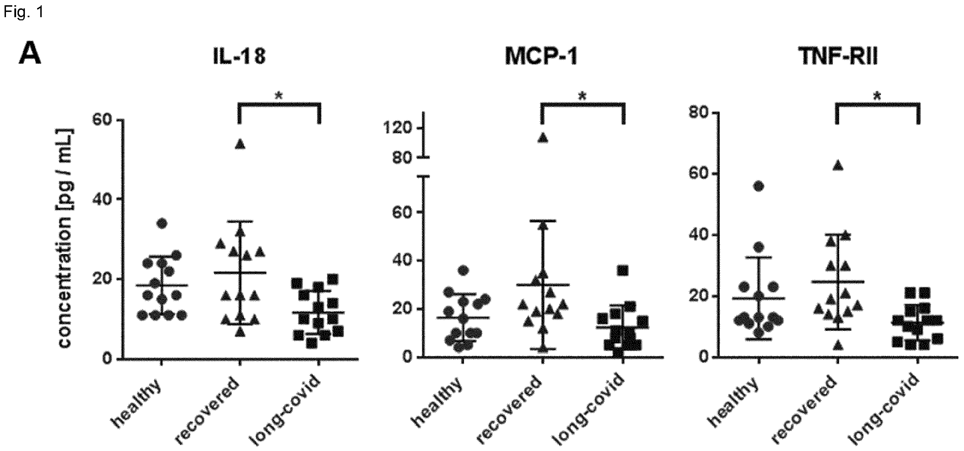

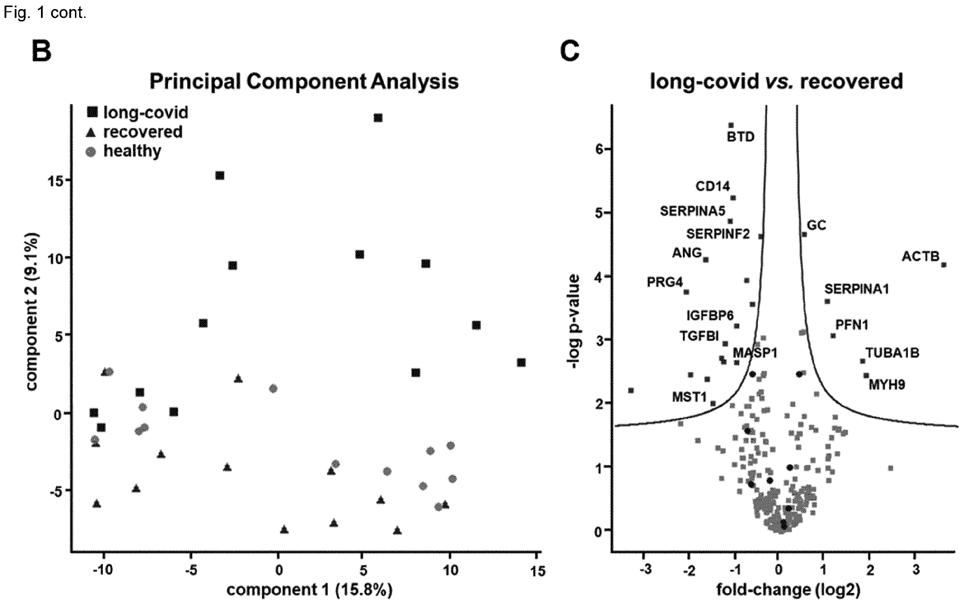

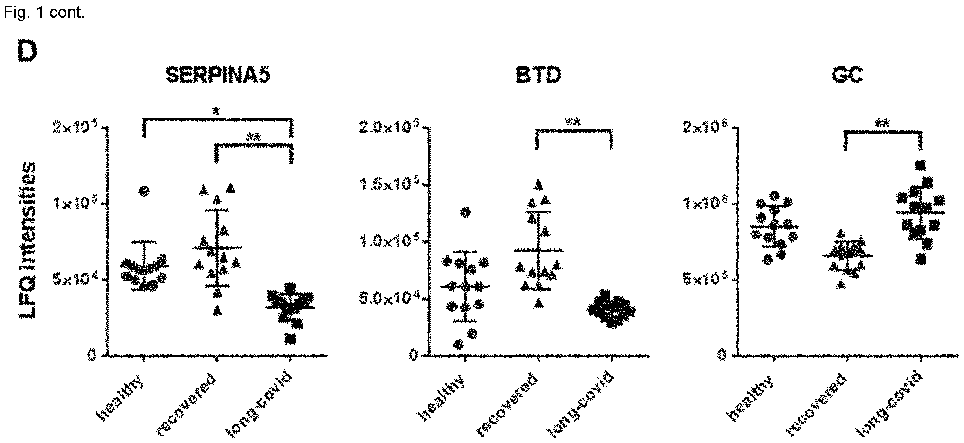

**Figure 1****: Cytokine and proteome profiles demonstrate a lack of pro-inflammatory activities in long-covid patients. (A)** Significant differences of IL-18 as well as the macrophage-derived cytokines MCP-1 and TNF-RII between long-covid and fully recovered patients are depicted. **(B)** A Principal Component Analysis based on proteome profiling of plasma samples from long-covid patients (square), fully recovered patients (triangle) and vaccinated healthy controls (grey circle) is shown. **(C)** Significant differences in plasma protein abundance between long-covid and fully recovered patients are visualized by a volcano plot. **(D)** Label-free quantification (LFQ) intensities derived from untargeted plasma proteomics of SERPINA5, Biotinidase (BTD) and Vitamin D-binding protein (GC) are depicted for all study groups. * p ≤ 0.05, ** p ≤ 0.01

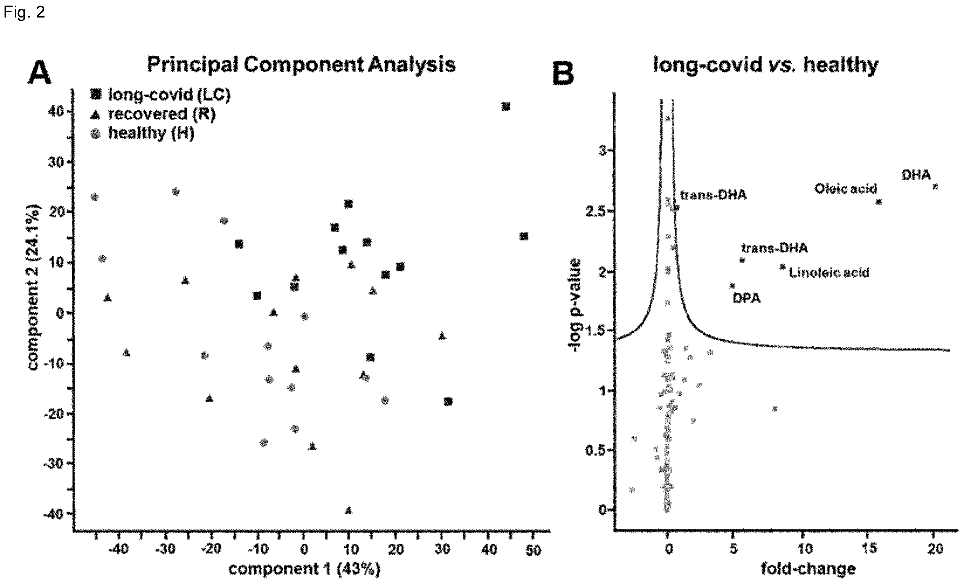

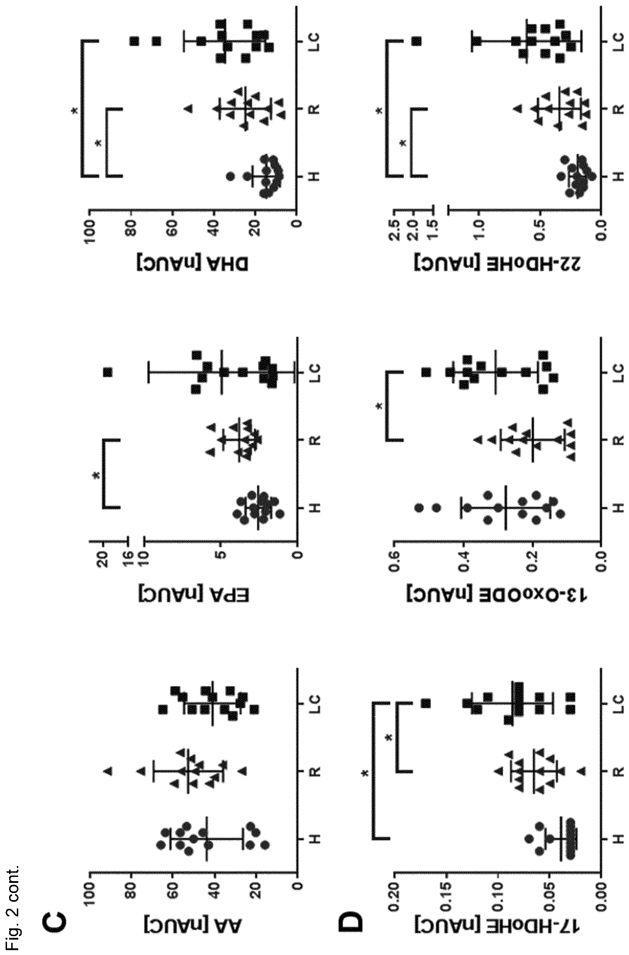

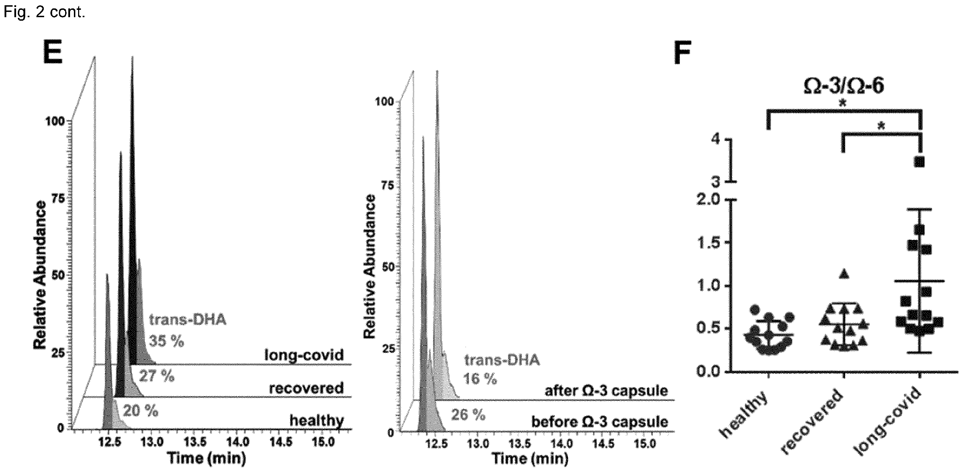

**Figure 2****: Increased levels of fatty acids with a prevalence for Q3-fatty acids and anti-inflammatory docosanoids is characteristic for long-covid. (A)** A Principal Component Analysis based on eicosanoid analysis of plasma samples from long-covid patients (square), fully recovered patients (triangle) and vaccinated healthy controls (grey circle) is shown. **(B)** Significant differences in plasma eicosanoids between long-covid patients and vaccinated healthy controls are visualized by a volcano plot. **(C, D)** Normalized area under the curve (nAUC) values of arachidonic acid (AA), eicosapentaenoic acid (EPA), docosahexaenoic acid (DHA) and the eicosanoids 17-HDoHE, 13-OxoODE as well as 22-HDoHE are depicted for each study group (H, vaccinated healthy controls; R, fully recovered patients; LC, long-covid patients). **(E)** Increased levels of trans-DHA in long-covid patients are shown in an extracted ion chromatogram (m/z = 327.233 at a retention time (Time) of 12.5 min). No increase in trans-DHA levels after nutritional supplementation of omega-3 (Ω-3) capsules was observed in healthy controls. Percentage (%) of trans-DHA to DHA is depicted in grey for each study group. **(F)** A significant increase in the omega-3 to omega-6 ratio (Ω-3/ Ω-6) was observed in long-covid patients compared to vaccinated healthy controls as well as fully recovered patients. * p ≤ 0.05

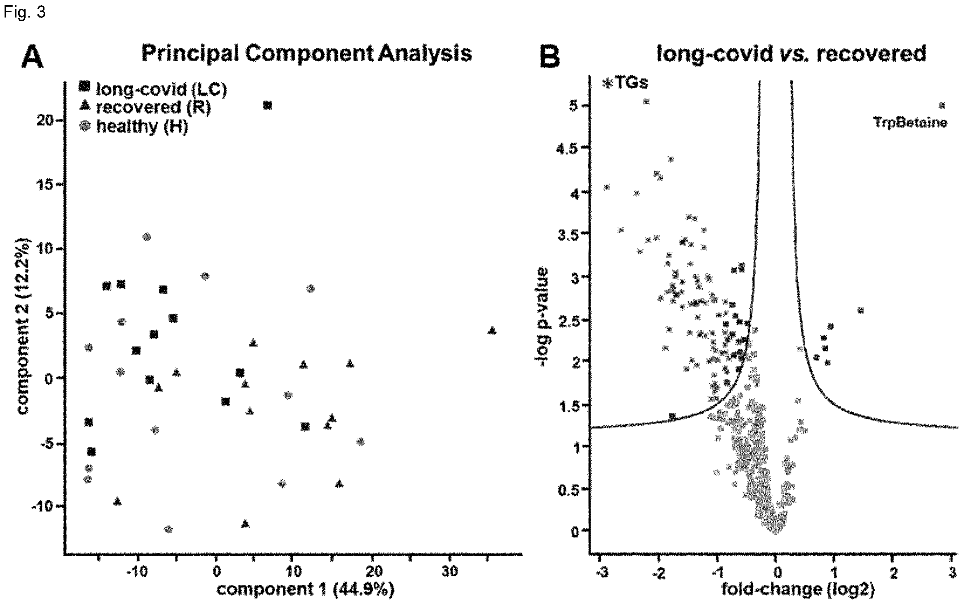

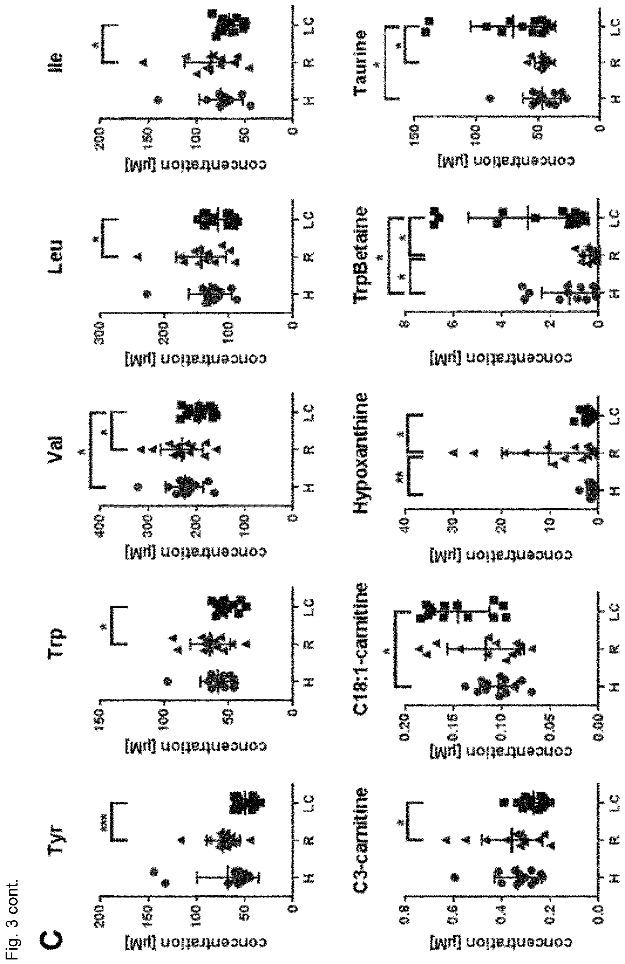

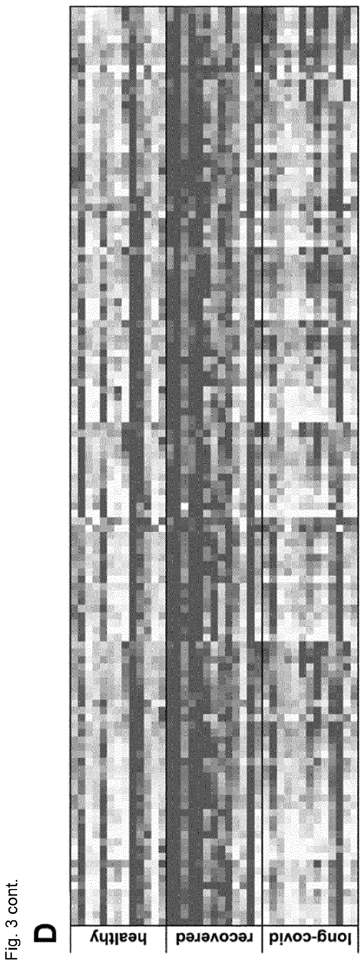

**Figure 3****: Plasma metabolomics reveals disturbances in the energy metabolism of long-covid patients. (A)** A Principal Component Analysis based on metabolomic analysis of plasma samples from long-covid patients (square), fully recovered patients (triangle) and vaccinated healthy controls (grey circle) is shown. **(B)** Significant higher levels of TrpBetaine as well as significant lower levels of triacylglycerols (TGs, star) in plasma samples of long-covid patients are visualized by a volcano plot. **(C)** Plasma levels of the amino acids tyrosine (Tyr), tryptophan (Trp), valine (Val), leucine (Leu) and isoleucine (lie) as well as of carnitines, hypoxanthine, TrpBetaine and taurine are depicted for each study group (H, vaccinated healthy controls; R, fully recovered patients; LC, long-covid patients). **(D)** A heat map of all identified triacylglycerols (TGs) displays higher levels of TGs in fully recovered patients compared to long-covid patients and vaccinated healthy controls. * p ≤ 0.05, ** p ≤ 0.01, *** p ≤ 0.001

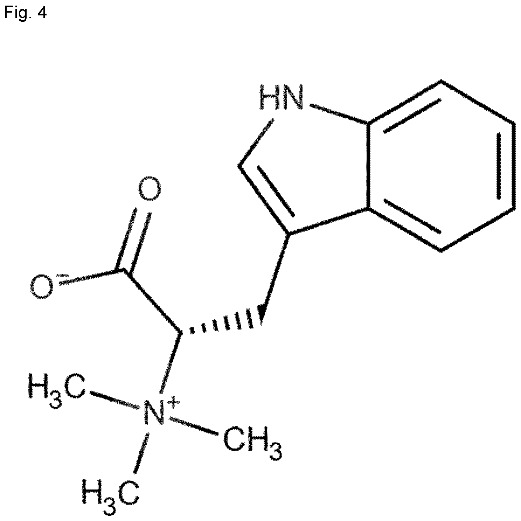

**Figure 4****:** Chemical structure of tryptophan betaine.

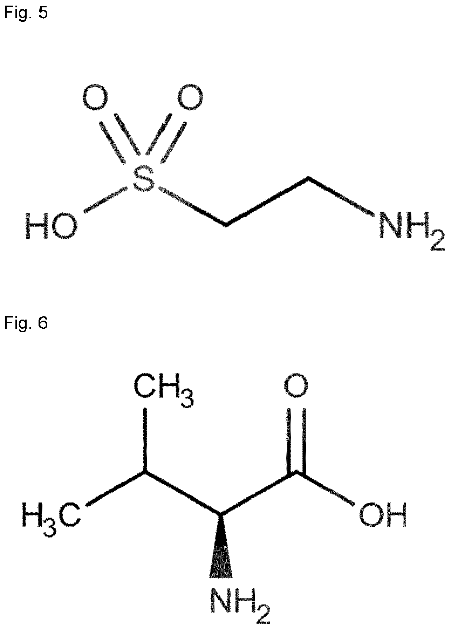

**Figure 5****:** Chemical structure of taurine.

**Figure 6:** Chemical structure of valine.

### DETAILED DESCRIPTION

Unless indicated or defined otherwise, all terms used herein have their usual meaning in the art, which will be clear to the skilled person. Reference is for example made to the standard handbooks, such as Sambrook et al, "Molecular Cloning: A Laboratory Manual" (4th Ed.), Vols. 1 -3, Cold Spring Harbor Laboratory Press (2012); Krebs et al., "Lewin's Genes XI", Jones & Bartlett Learning, (2017), and Murphy & Weaver, "Janeway's Immunobiology" (9th Ed., or more recent editions), Taylor & Francis Inc, 2017.

The subject matter of the claims specifically refers to artificial products or methods employing or producing such artificial products, which may be variants of native (wild-type) products. Though there can be a certain degree of sequence identity to the native structure, it is well understood that the materials, methods and uses of the invention, e.g., specifically referring to isolated nucleic acid sequences, amino acid sequences, fusion constructs, expression constructs, transformed host cells and modified proteins, are "man-made" or synthetic, and are therefore not considered as a result of "laws of nature".

The terms "comprise", "contain", "have" and "include" as used herein can be used synonymously and shall be understood as an open definition, allowing further members or parts or elements. "Consisting" is considered as a closest definition without further elements of the consisting definition feature. Thus "comprising" is broader and contains the "consisting" definition.

The term "about" as used herein refers to the same value or a value differing by +/-5 % of the given value.

As used herein and in the claims, the singular form, for example "a", "an" and "the" includes the plural, unless the context clearly dictates otherwise.

As used herein, amino acids refer to twenty naturally occurring amino acids encoded by sixty-one triplet codons. These 20 amino acids can be split into those that have neutral charges, positive charges, and negative charges:
The "neutral" amino acids are shown below along with their respective three-letter and single-letter code and polarity: Alanine (Ala, A; nonpolar, neutral), Asparagine (Asn, N; polar, neutral), Cysteine (Cys, C; nonpolar, neutral), Glutamine (Gin, Q; polar, neutral), Glycine (Gly, G; nonpolar, neutral), Isoleucine (lie, I; nonpolar, neutral), Leucine (Leu, L; nonpolar, neutral), Methionine (Met, M; nonpolar, neutral), Phenylalanine (Phe, F; nonpolar, neutral), Proline (Pro, P; nonpolar, neutral), Serine (Ser, S; polar, neutral), Threonine (Thr, T; polar, neutral), Tryptophan (Trp, W; nonpolar, neutral), Tyrosine (Tyr, Y; polar, neutral), Valine (Val, V; nonpolar, neutral), and Histidine (His, H; polar, positive (10%) neutral (90%)).

The "positively" charged amino acids are: Arginine (Arg, R; polar, positive), and Lysine (Lys, K; polar, positive).

The "negatively" charged amino acids are: Aspartic acid (Asp, D; polar, negative), and Glutamic acid (Glu, E; polar, negative).

As used herein, "post-viral fatigue syndrome" refers to a condition characterized by patients complaining of exhaustion, fatigue, muscle aches and pains, and invariable psychiatric symptoms such as emotional lability, poor memory/concentration, and depression. Symptoms of post-viral fatigue syndrome may begin suddenly after an acute viral infection and may commonly include incapacitating and persistent fatigue, muscle aches, joint pains, weakness after exercise, headaches, swollen glands, digestive disorders, inability to concentrate, memory loss, recurring minor infections or low-grade fevers, depression, an increasing sense of being unable to function, sleep disturbance, light sensitivity, food intolerance and environmental allergies. Post-viral fatigue syndrome is also called myalgic encephalomyelitis and is a common disorder, which has been known previously under a variety of different names, i.e., Iceland disease or Royal Free disease. It may occur in epidemics or sporadically.

According to one embodiment of the invention, "post-viral fatigue syndrome" may be "chronic post-viral fatigue syndrome".

Post-viral fatigue syndrome may be caused by any virus capable of entering the human body. Non-limiting examples of such a virus capable of causing post-viral fatigue syndrome are SARS-CoV, such as SARS-CoV-2 and COVID-19, Epstein-Barr virus, Human Herpes virus, human immunodeficiency virus, enterovirus, rubella, west nile virus, influenza virus, and ross river virus.

According to a specific embodiment, the post-viral fatigue syndrome is chronic post-COVID-19 syndrome or long-COVID.

As used herein, the terms "long-COVID" or "long-COVID syndrome" may be used interchangeably.

Chronic post-COVID-19 syndrome or long-COVID has been described as a condition with continuing breathlessness and fatigue even months after the onset of the SARS-CoV-2 virus induced disease. According to Bornstein S.R. et al. (2022) there are striking similarities to myalgic encephalomyelitis also called chronic fatigue syndrome linked to a viral and autoimmune pathogenesis.

The disease state of acute COVID-19 infections is characterized by pro-inflammatory activities. Thus, a failure to clear such inflammatory activities was commonly assumed to account for long-COVID symptoms. The inventors of the present invention have surprisingly found that rather the opposite of an inflammatory state, namely a systemic anti-inflammation, appears to be the main characteristic feature of long-COVID.

Surprisingly, the inventors of the present invention have found evidence for anti-inflammatory conditions prevalent in long-COVID patients. A lack of pro-inflammatory activities and the predominance of anti-inflammatory mediators in blood plasma was independently observed at the levels of cytokines, acute phase proteins, oxylipins and metabolites such as osmolytes.

According to one embodiment of the invention, the methods described herein comprise the determination of the production of a marker in a blood sample.

According to a specific embodiment, the methods described herein comprise the determination of the level of the marker(s).

According to a specific embodiment, in the methods described herein the level of the marker(s) are compared to a predetermined reference level or a reference sample.

As used herein, the term "marker" or "biomarker" refers to a measurable indicator of a biological state or condition. The marker may be a nucleotide, a protein, or a metabolite.

The markers further described herein are the following:
SERPINA5:
   Alternative name: serpin family A member 5; serine protease inhibitor
   Amino acid sequence of SERPINA5 (Uniprot: >sp|P05154|IPSP_HUMAN Plasma serine protease inhibitor OS=Homo sapiens OX=9606 GN=SERPINA5 PE=1 SV=3): 
MQLFLLLCLVLLSPQGASLHRHHPREMKKRVEDLHVGATVAPSSRRDFTFDLYRALA
SAAPSQSIFFSPVSISMSLAMLSLGAGSSTKMQILEGLGLNLQKSSEKELHRGFQQLL
QELNQPRDGFQLSLGNALFTDLVVDLQDTFVSAMKTLYLADTFPTNFRDSAGAMKQI
NDYVAKQTKGKIVDLLKNLDSNAVVIMVNYIFFKAKWETSFNHKGTQEQDFYVTSETV
VRVPMMSREDQYHYLLDRNLSCRVVGVPYQGNATALFILPSEGKMQQVENGLSEKT
LRKWLKMFKKRQLELYLPKFSIEGSYQLEKVLPSLGISNVFTSHADLSGISNHSNIQVS
EMVHKAVVEVDESGTRAAAATGTIFTFRSARLNSQRLVFNRPFLMFIVDNNILFLGKV
NRP (SEQ ID NO: 1)
   SERPINA5 is the physiologic inhibitor of furin, a protease essential for covid virulence. SERPINA5 is a heparin-dependent serine protease inhibitor acting in body fluids and secretions. It inactivates serine proteases by binding irreversibly to their serine activation site. It is involved in the regulation of intravascular and extravascular proteolytic activities and plays hemostatic roles in the blood plasma.
Tryptophan-betaine:
   Alternative name: Lenticin; Hypaphorine; L-Hypaphorine; L-Tryptophan betaine
   IUPAC name: (2S)-3-(1H-indol-3-yl)-2-(trimethylazaniumyl)propanoate
   The chemical structure of tryptophan-betaine is shown in Figure 4.
   Tryptophan-betaine is an amino acid betaine obtained by exhaustive methylation of the alpha-amino group of L-tryptophan with concomitant deprotonation of the carboxy group. It is an amino-acid betaine, a L-tryptophan derivative and an indole alkaloid.
Taurine:
   Alternative name: L-Taurine; 2-Aminoethanesulfonic acid
   IUPAC name: 2-aminoethane-1-sulfonic acid
   The chemical structure of taurine is shown in Figure 5.
   Taurine is an amino sulfonic acid that is the 2-amino derivative of ethanesulfonic acid. It is a naturally occurring amino acid derived from methionine and cysteine metabolism. It has a role as a human metabolite, as an antioxidant, a glycine receptor agonist and a radical scavenger.
Valine:
   Alternative name: L-Valine; L-alpha-Amino-beta-methylbutyric acid; (S)-2-Amino-3-methylbutyric acid; (S)-Valine
   IUPAC name: (2S)-2-amino-3-methylbutanoic acid
   The chemical structure of valine is shown in Figure 6.
   Valine is an aliphatic and extremely hydrophobic essential amino acid in humans related to leucine, valine is found in many proteins, mostly in the interior of globular proteins helping to determine three-dimensional structure. A glycogenic amino acid, valine maintains mental vigor, muscle coordination, and emotional calm.
Biotinidase (BTD):
   Amino acid sequence of biotinidase (Uniprot: >sp|P43251|BTD_HUMAN Biotinidase OS=Homo sapiens OX=9606 GN=BTD PE=1 SV=2): 
MAHAHIQGGRRAKSRFVVCIMSGARSKLALFLCGCYVVALGAHTGEESVADHHEAEY
YVAAVYEHPSILSLNPLALISRQEALELMNQNLDIYEQQVMTAAQKDVQIIVFPEDGIH
GFNFTRTSIYPFLDFMPSPQVVRWNPCLEPHRFNDTEVLQRLSCMAIRGDMFLVANL
GTKEPCHSSDPRCPKDGRYQFNTNVVFSNNGTLVDRYRKHNLYFEAAFDVPLKVDLI
TFDTPFAGRFGIFTCFDILFFDPAIRVLRDYKVKHVVYPTAWMNQLPLLAAIEIQKAFAV
AFGINVLAANVHHPVLGMTGSGIHTPLESFWYHDMENPKSHLIIAQVAKNPVGLIGAE
NATGETDPSHSKFLKILSGDPYCEKDAQEVHCDEATKWNVNAPPTFHSEMMYDNFT
LVPVWGKEGYLHVCSNGLCCYLLYERPTLSKELYALGVFDGLHTVHGTYYIQVCALV
RCGGLGFDTCGQEITEATGIFEFHLWGNFSTSYIFPLFLTSGMTLEVPDQLGWENDH
YFLRKSRLSSGLVTAALYGRLYERD (SEQ ID NO: 2).
   Biotinidase (BTD) is catalyzing the release of biotin from biocytin, the product of biotin-dependent carboxylases degradation. It is thus important for the recycling of biotin in the human body.
Vitamin D binding protein:
   Alternative names: Gc protein-derived macrophage activating factor, Gc-globulin, Group-specific component, Vitamin D-binding protein-macrophage activating factor.
   Amino acid sequence of vitamin D binding protein (Uniprot: >sp|P02774|VTDB_HUMAN Vitamin D-binding protein OS=Homo sapiens OX=9606 GN=GC PE=1 SV=2): 
MKRVLVLLLAVAFGHALERGRDYEKNKVCKEFSHLGKEDFTSLSLVLYSRKFP
SGTFEQVSQLVKEVVSLTEACCAEGADPDCYDTRTSALSAKSCESNSPFPVHPGTAE
CCTKEGLERKLCMAALKHQPQEFPTYVEPTNDEICEAFRKDPKEYANQFMWEYSTN
YGQAPLSLLVSYTKSYLSMVGSCCTSASPTVCFLKERLQLKHLSLLTTLSNRVCSQYA
 
AYGEKKSRLSNLIKLAQKVPTADLEDVLPLAEDITNILSKCCESASEDCMAKELPEHTV
KLCDNLSTKNSKFEDCCQEKTAMDVFVCTYFMPAAQLPELPDVELPTNKDVCDPGN
TKVMDKYTFELSRRTHLPEVFLSKVLEPTLKSLGECCDVEDSTTCFNAKGPLLKKELS
SFIDKGQELCADYSENTFTEYKKKLAERLKAKLPDATPTELAKLVNKHSDFASNCCSIN
SPPLYCDSEIDAELKNIL (SEQ ID NO: 3)
   Vitamin D binding protein (GC) is involved in vitamin D transport and storage, scavenging of extracellular G-actin, enhancement of the chemotactic activity of C5 alpha for neutrophils in inflammation and macrophage activation.

According to the invention, a method is provided for predicting post-viral fatigue syndrome in a human subject comprising determining the production of SERPINA5 in a blood sample of said subject, wherein a reduced production level of SERPINA5 is indicative of post-viral fatigue syndrome.

As used herein, "predicting" or "prediction" relates to the prediction of the possibility and risk of the affected disease or the development of such diseases in an individual.

As used herein, "indicative" or "indicate" e.g., in the context of indicating an event, such as a disease condition, the risk of a disease condition, disease progression, or treatment success is herein understood as a measure of risk and/or likelihood. Preferably, "indicating" of the presence or absence of an event is intended as a risk assessment, and is typically not to be construed in a limiting way as to point definitively to the absolute presence or absence of said event.

According to the invention, there is further provided a method for diagnosing post-viral fatigue syndrome in a human subject comprising determining the production or the production level of SERPINA5 in a blood sample of said subject, wherein a reduced production or production level is indicative of post-viral fatigue syndrome.

As used herein, "diagnosing" or "diagnosis" relates to the recognition and (early) detection of a clinical condition of a subject linked to post-viral fatigue syndrome.

"Prognosis" relates to the prediction of an outcome or a specific risk for a subject. This may also include an estimation of the chance of recovery or the chance of a disease progression for said subject.

According to a specific embodiment, a reduced production or production level of SERPINA5 is indicative of post-viral fatigue syndrome if the production or production level of SERPINA5 is reduced by 5, 10, 15, 20, 25, 30, 35, 40, 45, 50, 55, 60, 65, 70, 75, 80, 85, 90, or 95 %, preferably by at least 10, 20, 30, 40, 50, 60 % or even more, more preferably by 30 % compared to a predetermined reference value.

According to a specific embodiment, the method for predicting or the method of diagnosing post-viral fatigue syndrome described herein further comprises determining the production of at least one, at least two, at least three, at least four, or all of the marker(s) selected from tryptophan-betaine, taurine, valine, biotinidase, vitamin D binding protein, and any combination thereof.

According to a specific embodiment, the method for predicting or the method of diagnosing post-viral fatigue syndrome comprises determining the production of SERPINA5 and tryptophan-betaine, wherein a reduced production level of SERPINA5 and an increased production level of tryptophan-betaine is indicative of post-viral fatigue syndrome.

According to a specific embodiment, an increased production level of tryptophan-betaine is indicative of post-viral fatigue syndrome if the production level of tryptophan-betaine is increased 5, 10, 15, 20, 25, 30, 35, 40, 45, 50, 55, 60, 65, 70, 75, 80, 85, 90, or 95 %, preferably by at least 50, 60, 70, 75, 80, 85, 90, 95, 100 % or even more, more preferably by 100 % or even more compared to a predetermined reference value. Specifically, the production level of tryptophan-betaine may be increased 2-fold, 3-fold, 4-fold, or more, compared to a predetermined reference value.

According to a specific embodiment, the method for predicting or the method of diagnosing post-viral fatigue syndrome comprises determining the production of SERPINA5 and taurine, wherein a reduced production level of SERPINA5 and an increased production level of taurine is indicative of post-viral fatigue syndrome.

According to a specific embodiment, an increased production level of taurine is indicative of post-viral fatigue syndrome if the production level of taurine is increased by 5, 10, 15, 20, 25, 30, 35, 40, 45, 50, 55, 60, 65, 70, 75, 80, 85, 90, 95, 100 % or even more, preferably by at least 10, 20, 30, 40, 50, 60 % or even more, more preferably by 50 % compared to a predetermined reference value. Specifically, the production level of taurine may be increased 2-fold, 3-fold, 4-fold, or more, compared to a predetermined reference value.

According to a specific embodiment, the method for predicting or the method of diagnosing post-viral fatigue syndrome comprises determining the production of SERPINA5 and valine, wherein a reduced production level of SERPINA5 and a reduced production level of valine is indicative of post-viral fatigue syndrome.

According to a specific embodiment, a reduced production level of valine is indicative of post-viral fatigue syndrome if the production level of valine is reduced by 5, 10, 15, 20, 25, 30, 35, 40, 45, 50, 55, 60, 65, 70, 75, 80, 85, 90, 95, 100 % or even more, preferably by at least 10, 20, 30, 40, 50, 60 % or even more, more preferably by 10 % compared to a predetermined reference value.

According to a specific embodiment, the method for predicting or the method of diagnosing post-viral fatigue syndrome comprises determining the production of SERPINA5 and biotinidase, wherein a reduced production level of SERPINA5 and a reduced production level of biotinidase is indicative of post-viral fatigue syndrome.

According to a specific embodiment, a reduced production level of biotinidase BTD is indicative of post-viral fatigue syndrome if the production level of biotinidase BTD is reduced by 5, 10, 15, 20, 25, 30, 35, 40, 45, 50, 55, 60, 65, 70, 75, 80, 85, 90, 95, 100 % or even more, preferably by at least 10, 20, 25, 30, 40, 50, 60 % or even more, more preferably by 25 % compared to a predetermined reference value.

According to a specific embodiment, the method for predicting or the method of diagnosing post-viral fatigue syndrome comprises determining the production of SERPINA5 and vitamin D binding protein, wherein a reduced production level of SERPINA5 and an increased production level of vitamin D binding protein is indicative of post-viral fatigue syndrome.

According to a specific embodiment, an increased production level of vitamin D binding protein is indicative of post-viral fatigue syndrome if the production level of vitamin D binding protein is increased by 5, 10, 15, 20, 25, 30, 35, 40, 45, 50, 55, 60, 65, 70, 75, 80, 85, 90, 95, 100 % or even more, preferably by at least 10, 20, 30, 40, 50, 60 % or even more, more preferably by 10 % compared to a predetermined reference value. Specifically, the production level of vitamin D binding protein may be increase 2-fold, 3-fold, 4-fold, or more compared to a predetermined reference value.

According to a specific embodiment, the method for predicting or the method of diagnosing post-viral fatigue syndrome comprises determining the production of SERPINA5, tryptophan-betaine, taurine, valine, biotinidase, and vitamin D binding protein, wherein reduced production of SERPINA5; increased production of tryptophan-betaine; increased production of taurine; reduced production of valine; reduced production of biotinidase; and increased production of vitamin D binding protein is indicative of post-viral fatigue syndrome.

According to a specific embodiment, in the method for predicting or in the method of diagnosing post-viral fatigue syndrome described herein, the subject receives a standard treatment of post-viral fatigue syndrome, chronic fatigue syndrome, chronic post-COVID-19 syndrome, or long-COVID. Such a standard treatment may include painkillers and antidepressants together with various supplements such as vitamins, but also addresses on lifestyle parameters and physical activities.

According to a specific embodiment, in the method for predicting or in the method of diagnosing post-viral fatigue syndrome described herein, the subject is monitored by determining and comparing the production of the marker(s) at different time points, preferably wherein the blood samples are drawn at least twice, at two or more different time points. Specifically, samples may be drawn at different time points to provide for comparability or the results, such as the first and second time points referred to herein, are particularly of the same type, and treated the same way.

According to the invention, a method is provided for monitoring treatment of a post-viral fatigue syndrome in a patient comprising determining the production of SERPINA5 in a blood sample of said patient, wherein increase of the production of SERPINA5 is indicative of the patient's response to said treatment.

As used herein, the term "monitoring" or "monitoring treatment" relates to the use of a method described herein for monitoring, therapy monitoring, therapy guidance and/or therapy control. "Monitoring" relates to keeping track of subject such as a post-viral fatigue syndrome patient and potentially occurring disease progression or complications, e.g., to analyze the progression of the healing process or the influence of a particular treatment or therapy on the health state of the patient. Specifically, the patient is monitored by determining and comparing the production level of a marker as described herein at different timepoints.

According to a specific embodiment, increase of the production of SERPINA5 is indicative of the patient's response to the treatment if the production level of SERPINA5 is increased by 1, 2, 3, 4, 5, 6, 7, 8, 9, 10, 15, 20, 25, 30, 35, 40, 45, 50, 55, 60, 65, 70, 75, 80, 85, 90, or 95 %, preferably by at least 10, 20, 30, 40, 50, 60 % or even more, more preferably by 20 % compared to a reference sample. Specifically, increase of the production level of SERPINA5 may be indicative of the patient's response to the treatment if the production level of SERPINA5 is increased 2-fold, 3-fold, 4-fold, or more compared to a reference sample. Alternatively, increase of the production level of SERPINA5 is indicative of the patient's response to the treatment if the production level of SERPINA5 is normalized. Alternatively, increase of the production level of SERPINA5 is indicative of the patient's response to the treatment if the production level of SERPINA5 is approaching a predetermined reference value.

According to a specific embodiment, the method for monitoring treatment of a post-viral fatigue syndrome in a patient further comprises determining the production of at least one, at least two, at least three, at least four, or all of the marker(s) selected from tryptophan-betaine, taurine, valine, biotinidase, vitamin D binding protein, and any combination thereof.

According to a specific embodiment, the method for monitoring treatment of a post-viral fatigue syndrome in a patient comprises determining the production of SERPINA5 and tryptophan-betaine, wherein an increased production level of SERPINA5 and a reduced production level of tryptophan-betaine is indicative of the patient's response to said treatment.

According to a specific embodiment, an reduced production level of tryptophan-betaine is indicative of the patient's response to said treatment if the production level of tryptophan-betaine is reduced 1, 2, 3, 4, 5, 6, 7, 8, 9, 10, 15, 20, 25, 30, 35, 40, 45, 50, 55, 60, 65, 70, 75, 80, 85, 90, or 95 %, preferably by at least 10, 20, 30, 40, 50, 60 % or even more, more preferably by **50** % compared to a reference sample. Alternatively, increase of the production of SERPINA5 is indicative of the patient's response to the treatment if the production level of SERPINA5 is normalized. Alternatively, increase of the production of SERPINA5 is indicative of the patient's response to the treatment if the production level of SERPINA5 is approaching a predetermined reference value.

According to a specific embodiment, the method for monitoring treatment of a post-viral fatigue syndrome in a patient comprises determining the production of SERPINA5 and taurine, wherein an increased production level of SERPINA5 and a reduced production level of taurine is indicative of the patient's response to said treatment.

According to a specific embodiment, a reduced production level of taurine is indicative of the patient's response to said treatment if the production level of taurine is reduced by 1, 2, 3, 4, 5, 6, 7, 8, 9, 10, 15, 20, 25, 30, 35, 40, 45, 50, 55, 60, 65, 70, 75, 80, 85, 90, or 95 %, preferably by at least 10, 20, 30, 40, 50, 60 % or even more, more preferably by **25** % compared to a reference sample. Alternatively, reduction of the production level of taurine is indicative of the patient's response to the treatment if the production level of taurine is normalized. Alternatively, reduction of the production level of taurine is indicative of the patient's response to the treatment if the production level of taurine is approaching a predetermined reference value.

According to a specific embodiment, the method for monitoring treatment of a post-viral fatigue syndrome in a patient comprises determining the production of SERPINA5 and valine, wherein an increased production level of SERPINA5 and an increased production level of valine is indicative of the patient's response to said treatment.

According to a specific embodiment, an increased production level of valine is indicative of the patient's response to said treatment if the production level of valine is increased by 1, 2, 3, 4, 5, 6, 7, 8, 9, 10, 15, 20, 25, 30, 35, 40, 45, 50, 55, 60, 65, 70, 75, 80, 85, 90, or 95 %, preferably by at least 1, 2, 3, 4, 5, 6, 7, 8, 9, 10, 20, 30, 40, 50, 60 % or even more, more preferably by **5** % compared to a reference sample. Specifically, increase of the production level of valine may be indicative of the patient's response to the treatment if the production level of valine is increased 2-fold, 3-fold, 4-fold, or more compared to a reference sample. Alternatively, increase of the production level of valine is indicative of the patient's response to the treatment if the production level of valine is normalized. Alternatively, increase of the production level of valine is indicative of the patient's response to the treatment if the production level of valine is approaching a predetermined reference value.

According to a specific embodiment, the method for monitoring treatment of a post-viral fatigue syndrome in a patient comprises determining the production of SERPINA5 and biotinidase, wherein an increased production level of SERPINA5 and an increased production level of biotinidase is indicative of the patient's response to said treatment.

According to a specific embodiment, an increased production level of biotinidase is indicative of the patient's response to said treatment if the production level of biotinidase is increased by 1, 2, 3, 4, 5, 6, 7, 8, 9, 10, 15, 20, 25, 30, 35, 40, 45, 50, 55, 60, 65, 70, 75, 80, 85, 90, or 95 %, preferably by at least 1, 2, 3, 4, 5, 6, 7, 8, 9, 10, 20, 30, 40, 50, 60 % or even more, more preferably by 10 % compared to a reference sample. Specifically, increase of the production level of biotinidase may be indicative of the patient's response to the treatment if the production level of biotinidase is increased 2-fold, 3-fold, 4-fold, or more compared to a reference sample. Alternatively, increase of the production level of biotinidase is indicative of the patient's response to the treatment if the production level of biotinidase is normalized. Alternatively, increase of the production level of biotinidase is indicative of the patient's response to the treatment if the production level of biotinidase is approaching a predetermined reference value.

According to a specific embodiment, the method for monitoring treatment of a post-viral fatigue syndrome in a patient comprises determining the production of SERPINA5 and vitamin D binding protein, wherein an increased production level of SERPINA5 and a reduced production level of vitamin D binding protein is indicative of the patient's response to said treatment.

According to a specific embodiment, a reduced production level of vitamin D binding protein is indicative of the patient's response to said treatment if the production level of vitamin D binding protein is reduced by 1, 2, 3, 4, 5, 6, 7, 8, 9, 10, 15, 20, 25, 30, 35, 40, 45, 50, 55, 60, 65, 70, 75, 80, 85, 90, or 95 %, preferably by at least 1, 2, 3, 4, 5, 6, 7, 8, 9, 10, 20, 30, 40, 50, 60 % or even more, more preferably by **5** % compared to a reference sample. Alternatively, reduction of the production level of vitamin D binding protein is indicative of the patient's response to the treatment if the production level of vitamin D binding protein is normalized. Alternatively, reduction of the production level of vitamin D binding protein is indicative of the patient's response to the treatment if the production level of vitamin D binding protein is approaching a predetermined reference value.

According to a specific embodiment, the method for monitoring treatment of a post-viral fatigue syndrome in a patient comprises determining the production of SERPINA5, tryptophan-betaine, taurine, valine, biotinidase, and vitamin D binding protein, wherein increased production of SERPINA5; reduced production of tryptophan-betaine; reduced production of taurine; increased production of valine; increased production of biotinidase; and reduced production of vitamin D binding protein is indicative of the patient's response to said treatment.

According to a specific embodiment, in method for monitoring treatment of a post-viral fatigue syndrome in a patient described herein, the subject receives a standard treatment of post-viral fatigue syndrome, chronic fatigue syndrome, chronicpost-COVID-19 syndrome, or long-COVID. Such a standard treatment may include painkillers and antidepressants together with various supplements such as vitamins, but also addresses on lifestyle parameters and physical activities.

According to a specific embodiment, in method for monitoring treatment of a post-viral fatigue syndrome in a patient described herein, the subject is monitored by determining and comparing the production of the marker(s) at different time points, preferably wherein the blood samples are drawn at least twice, at two or more different time points. Specifically, samples may be drawn at different time points to provide for comparability or the results, such as the first and second time points referred to herein, are particularly of the same type, and treated the same way.

According to the invention, a method is provided for identifying a patient eligible for treatment of post-viral fatigue syndrome comprising determining the production of SERPINA5 in a blood sample of said patient, wherein a reduced production level is identifying said patient being eligible for treatment.

The term "identifying a patient eligible for treatment" as used herein refers to the identification of a patient showing symptoms of post-viral fatigue syndrome to be eligible for treatment of post-viral fatigue syndrome.

According to a particular embodiment, the methods and markers described herein are used for therapy guidance, stratification, monitoring and/or control. To this end, the methods described herein specifically further comprise applying, maintaining, reducing, elevating or not applying a therapy based on whether the subject is at said risk of disease or disease progression.

According to a specific embodiment, a reduced production or production level of SERPINA5 is identifying said patient being eligible for treatment if the production or production level of SERPINA5 is reduced by 5, 10, 15, 20, 25, 30, 35, 40, 45, 50, 55, 60, 65, 70, 75, 80, 85, 90, or 95 %, preferably by at least 10, 20, 30, 40, 50, 60 % or even more, more preferably by 30 % compared to a predetermined reference value.

According to a specific embodiment, the method for identifying a patient eligible for treatment of post-viral fatigue syndrome described herein further comprises determining the production of at least one, at least two, at least three, at least four, or all of the marker(s) selected from tryptophan-betaine, taurine, valine, biotinidase, vitamin D binding protein, and any combination thereof.

According to a specific embodiment, the method for identifying a patient eligible for treatment of post-viral fatigue syndrome comprises determining the production of SERPINA5 and tryptophan-betaine, wherein a reduced production level of SERPINA5 and an increased production level of tryptophan-betaine is identifying said patient being eligible for treatment.

According to a specific embodiment, an increased production level of tryptophan-betaine is identifying said patient being eligible for treatment if the production level of tryptophan-betaine is increased by 5, 10, 15, 20, 25, 30, 35, 40, 45, 50, 55, 60, 65, 70, 75, 80, 85, 90, or 95 %, preferably by at least 50, 60, 70, 75, 80, 85, 90, 95, 100 % or even more, more preferably by 100 % or even more compared to a predetermined reference value. Specifically, the production level of tryptophan-betaine may be increased 2-fold, 3-fold, 4-fold, or more compared to a predetermined reference value.

According to a specific embodiment, the method for identifying a patient eligible for treatment of post-viral fatigue syndrome comprises determining the production of SERPINA5 and taurine, wherein a reduced production level of SERPINA5 and an increased production level of taurine is identifying said patient being eligible for treatment.

According to a specific embodiment, an increased production level of taurine is identifying said patient being eligible for treatment if the production level of taurine is increased by 5, 10, 15, 20, 25, 30, 35, 40, 45, 50, 55, 60, 65, 70, 75, 80, 85, 90, 95 or 100 % or even more, preferably by at least 10, 20, 30, 40, 50, 60 % or even more, more preferably by 50 % compared to a predetermined reference value. Specifically, the production level of taurine may be increased 2-fold, 3-fold, 4-fold, or more compared to a predetermined reference value.

According to a specific embodiment, the method for identifying a patient eligible for treatment of post-viral fatigue syndrome comprises determining the production of SERPINA5 and valine, wherein a reduced production level of SERPINA5 and a reduced production level of valine is identifying said patient being eligible for treatment.

According to a specific embodiment, a reduced production level of valine is identifying said patient being eligible for treatment if the production level of valine is reduced by 5, 10, 15, 20, 25, 30, 35, 40, 45, 50, 55, 60, 65, 70, 75, 80, 85, 90, 95, or 100 % or even more, preferably by at least 10, 20, 30, 40, 50, 60 % or even more, more preferably by 10 % compared to a predetermined reference value.

According to a specific embodiment, the method for identifying a patient eligible for treatment of post-viral fatigue syndrome comprises determining the production of SERPINA5 and biotinidase, wherein a reduced production level of SERPINA5 and a reduced production level of biotinidase is identifying said patient being eligible for treatment.

According to a specific embodiment, a reduced production level of biotinidase is identifying said patient being eligible for treatment if the production level of biotinidase is reduced by 5, 10, 15, 20, 25, 30, 35, 40, 45, 50, 55, 60, 65, 70, 75, 80, 85, 90, 95, or 100 % or even more, preferably by at least 10, 20, 25, 30, 40, 50, 60 % or even more, more preferably by 25 % compared to a predetermined reference value.

According to a specific embodiment, the method for identifying a patient eligible for treatment of post-viral fatigue syndrome comprises determining the production of SERPINA5 and vitamin D binding protein, wherein a reduced production level of SERPINA5 and an increased production level of vitamin D binding protein is identifying said patient being eligible for treatment.

According to a specific embodiment, an increased production level of vitamin D binding protein is identifying said patient being eligible for treatment if the production level of vitamin D binding protein is increased by 5, 10, 15, 20, 25, 30, 35, 40, 45, 50, 55, 60, 65, 70, 75, 80, 85, 90, 95, or 100 %, preferably by at least 5, 10, 20, 30, 40, 50, 60 % or even more, more preferably by 10 % compared to a predetermined reference value. Specifically, the production level of vitamin D binding protein may be increased 2-fold, 3-fold, 4-fold, or more compared to a predetermined reference value.

According to a specific embodiment, the method for identifying a patient eligible for treatment of post-viral fatigue syndrome comprises determining the production of SERPINA5, tryptophan-betaine, taurine, valine, biotinidase, and vitamin D binding protein, wherein reduced production of SERPINA5; increased production of tryptophan-betaine; increased production of taurine; reduced production of valine; reduced production of biotinidase; and increased production of vitamin D binding protein is identifying said patient being eligible for treatment.

According to a specific embodiment, in the method for identifying a patient eligible for treatment of post-viral fatigue syndrome described herein, the subject receives a standard treatment of post-viral fatigue syndrome, chronic fatigue syndrome, chronicpost-COVID-19 syndrome, or long-COVID. Such a standard treatment may include painkillers and antidepressants together with various supplements such as vitamins, but also addresses on lifestyle parameters and physical activities.

According to a specific embodiment, in the method for identifying a patient eligible for treatment of post-viral fatigue syndrome described herein, the subject is monitored by determining and comparing the production of the marker(s) at different time points, preferably wherein the blood samples are drawn at least twice, at two or more different time points. Specifically, samples may be drawn at different time points to provide for comparability of the results, such as the first and second time points referred to herein, are particularly of the same type, and treated the same way.

The terms "subject", "patient" or "individual" can be used interchangeably.

According to one embodiment, a subject or a patient is a human being.

The term "production" as used herein is meant to encompass all steps of anabolic and catabolic metabolism in the human body. Thereby, the term "production" as used herein is meant to encompass the steps of protein expression and protein degradation as well as enzymatic or chemical formation and degradation of metabolites. The term "expression" as used herein, is meant to encompass at least one step selected from the group consisting of DNA transcription into mRNA, mRNA processing, mRNA maturation, mRNA export, translation, protein folding, and protein transport. The term "protein degradation" as used herein is meant to encompass at least one step selected from proteasomal degradation, autophagic degradation or degradation caused by proteases. Thereby, "determining the production" as used herein refers to the determination of any of the foregoing groups.

According to a specific embodiment, production of a marker is determined by determining production products, such as those encoded by a marker gene, or those of the respective complementary sequence, including e.g., polypeptides, proteins, transcripts, like mRNA, or micro-RNA. The term "mRNA" as used herein shall refer to any of pre-mRNA transcript(s), transcript processing intermediates, mature mRNA(s) ready for translation and transcripts of the gene or genes, or nucleic acids derived from the mRNA transcript(s). A nucleic acid derived from an mRNA transcript is herein understood to refer to a nucleic acid for whose synthesis the mRNA transcript or a subsequence thereof has ultimately served as a template. A cDNA reverse transcribed from an mRNA, an RNA transcribed from that cDNA, a DNA amplified from the cDNA, an RNA transcribed from the amplified DNA, etc., are all derived from the mRNA transcript and detection of such derived products is indicative of the presence and/or abundance of the original transcript in a sample. Thus, mRNA derived samples include, but are not limited to, mRNA transcripts of the gene or genes, cDNA reverse transcribed from the mRNA, cRNA transcribed from the cDNA, DNA amplified from the genes, RNA transcribed from amplified DNA, and the like.

According to a specific embodiment, production of a marker is also determined by determining production of products of metabolic processes and metabolites. A "metabolite" is an intermediate or end product of metabolism. Specifically, the molecules taurine, tryptophane-betaine, and valine are herein considered as metabolites.

According to a specific embodiment, the protein production and/or nucleic acid production and/or metabolite production is determined, either qualitatively or quantitatively.

In a specific quantitative determination method, the production of the marker is normalized to the median production of one or more reference genes e.g., used as internal control.

According to a specific embodiment, specific markers as described herein are preferably determined by testing for the respective the markers, such as expressed polypeptides or proteins; polynucleotides or nucleic acid molecules, like mRNA; and/or metabolites such as amino acids or amino acid derivatives, e.g., taurine or tryptophan-betaine, indicative of marker production.

According to a specific embodiment, the markers s can be determined applying an analytical method selected from the group consisting of mass spectrometry (MS), immunoassay, luminescence immunoassay (LIA), radioimmunoassay (RIA), chemiluminescence- and fluorescence- immunoassays (e.g. immunofluorescence staining), enzyme immunoassay (EIA), Enzyme-linked immunoassays (ELISA), luminescence-based bead arrays, magnetic beads based arrays, protein microarray assays, rapid test formats e.g., immunochromatographic strip tests, and automated systems/analyzers.

According to a specific aspect, the level of a marker protein or a metabolite is determined using an immunoassay, such as an immunohistochemical assay, an immunoblotting assay, or a flow cytometry assay.

Specific analytical methods may employ a ligand which is an immunoagent, such as an immunoagent for use in an immunoassay.

Specifically, the immunoassay is selected from the group consisting of an enzymatic immunoassay, such as an ELISA (e.g. a sandwich ELISA), lateral flow immunochromatographic assay, fluorescent immunoassay, radioimmunoassay, and magnetic immunoassay.

According to a specific embodiment, the analytical method may employ detection of a label conjugated to the marker, preferably an enzymatic label, a fluorescent label, or a radioisotope label.

According to a specific embodiment, protein production is determined by immunoassays, mass spectrometry, and/or chromatography. Preferably, protein production is determined by immunoassays, or assays based on mass spectrometry hyphenated with chromatography. Said assays based on mass spectrometry hyphenated with chromatography may be targeted or untargeted.

According to a specific embodiment, the RNA- production is measured by any of a nucleic amplification method, hybridizing or sequencing method. The expression is optionally employing amplification methods, among them signal or nucleic acid amplification methods, such as RT-qPCR. Specifically, an expression product being a marker nucleic acid is measured by a real-time reverse transcriptase PCR assay or a nucleic acid microarray assay.

According to one embodiment, a blood sample is whole blood, serum, plasma, or a fraction of any of the foregoing.

According to one embodiment, determining the production relates to the determination of the production level. Specifically, the level of a marker is determined by suitable analytical methods and means.

According to a specific embodiment, production is determined quantitatively or semi-quantitatively.

As used herein, determination of the level of production "quantitatively" refers to the determination of the amount or concentration of the specific marker. Quantitative determination means to determine the absolute amount or concentration of the specific marker. Thereby, the quantitative expression may be performed by determination of the amount or concentration relative to a standard. For example, the determination of the production level of a specific marker used herein in µM is a quantitative determination of the production level of said marker.

As used herein, determination of the level of production "semi-quantitatively" refers to the determination of the production level with a lower precision than in quantitative determination. Specifically, semi-quantitative determination may be performed for a certain concentration range of the specific marker in which the determination of the production level e.g., the amount or concentration of said marker in the sample, is determined relative to a standard calibration of said marker. Semi-quantitative determination may also be performed for the determination if a specific marker is in a certain concentration range in the sample. For example, the determination of the production level of a specific marker used herein in "Label-Free-Quantification unit" (LFQ) is a semi-quantitative determination of the production level of said marker.

The term "level" is herein understood as the absolute or relative amount or concentration of a marker, the presence or absence of the marker, a range of amount or concentration of the marker, a minimum and/or maximum amount or concentration of the marker, a mean amount or concentration of the marker, and/or a median amount or concentration of the marker. Specific levels are provided as a value of the amount representing the concentration, and specifically expressed as weight/volume; w/v, or mol/volume e.g., µM, or expressed as fold change of the marker in the sample.

In a particular embodiment, production of SERPINA5 or the level of SERPINA5 is determined by antibody-based methods such as ELISA or indirect peptide analysis methods based on chromatographic methods combined with suitable detection methods such as mass spectrometry, including targeted or untargeted methods.

In a particular embodiment, production of tryptophan-betaine or the level of tryptophan-betaine is determined by antibody-based methods such as ELISA or chromatographic methods combined with suitable detection methods such as mass spectrometry, including targeted or untargeted methods.

In a particular embodiment, production of taurine or the level of taurine is determined by antibody-based methods such as ELISA or chromatographic methods combined with suitable detection methods such as mass spectrometry, including targeted or untargeted methods.

In a particular embodiment, production of valine or the level of valine is determined by antibody-based methods such as ELISA or chromatographic methods combined with suitable detection methods such as mass spectrometry, including targeted or untargeted methods.

In a particular embodiment, production of biotinidase or the level of biotinidase is determined by antibody-based methods such as ELISA or indirect peptide analysis methods based on chromatographic methods combined with suitable detection methods such as mass spectrometry, including targeted or untargeted methods.

In a particular embodiment, production of vitamin D binding protein or the level of vitamin D binding protein is determined by antibody-based methods such as ELISA or indirect peptide analysis methods based on chromatographic methods combined with suitable detection methods such as mass spectrometry, including targeted or untargeted methods.

According to a specific embodiment, the determined level of a marker is compared to a predetermined reference value.

As used herein, the "predetermined reference value" is a value resulting from calibrating the method against samples of asymptomatic fully recovered post-viral patients, healthy individuals, and/or healthy individuals vaccinated against the specific virus which causes the post-viral fatigue syndrome in the subject or patient of the method.

The term "healthy" or "healthy individual" as used herein refers to a individual not suffering from the herein described symptoms of post-viral fatigue syndrome, specifically of chronicpost-COVID-19 fatigue syndrome or long-COVID.

According to a specific embodiment, the "predetermined reference value" is a value resulting from calibrating the method against samples of asymptomatic fully recovered post-COVID-19 patients and/or healthy individuals vaccinated against SARS-COV-2.

As used herein, the term "reference sample" is used in the context of the method for monitoring treatment of a post-viral fatigue syndrome in a patient. Thereby, the "reference sample" refers to an earlier sample of said patient. The earlier sample may be drawn from the patient at any time point before the respective treatment has started. Blood samples of said patient may be analyzed for marker(s) production at different time points, preferably at two or more time points. Thereby, the determined production or production level of a marker is compared to the reference sample, i.e., to the earlier sample, for the analysis of the patient's response to the treatment.

According to an alternative embodiment, the methods described herein may be quantitative or semi-quantitative methods such as determining whether the biomarker level in the sample is above a reference or threshold level.

According to a specific embodiment, the predetermined reference value is a threshold, also understood as a cut-off value indicating a marker concentration for the respective risk or the severity of disease. The respective concentrations that determine the threshold values depend on multiple parameters such as the time point of sample isolation and the assay or detection used for determining the biomarker level in said sample.

A threshold level may distinguish between healthy subjects and diseased subjects, and/or between subjects at low risk of developing disease or disease progression and those subjects who are at a higher risk of developing disease or disease progression. Low risk subjects can be e.g., healthy subjects, or those with an early stage of disease and/or stable disease. High risk subjects can be e.g., at the onset of disease, or already suffering from disease.

As used herein, the term "cutoff value" particularly refers to a threshold value which distinguishes subjects who are suffering from a disease or disease condition from a population of patients and/or subjects who are not suffering from the disease or disease condition, and in particular distinguishes subjects who are at high risk of disease or disease progression from a population of patients and/or subjects who are not at such high risk of disease or disease progression.

The relevant reference levels can be determined by well-known methods e.g. based on extensive data which can be routinely obtained by comparing samples from diseased patients with healthy subjects or subjects not suffering from such disease. Such references are understood as population average levels, for example mean marker population values, whereby patients that are diagnosed as post-viral fatigue syndrome patient, may be compared to a control population, wherein the control group preferably comprises more than 10, 20, 30, 40, 50 or more subjects.

Appropriate normal reference levels of the marker may be determined by measuring levels of such marker in one or more appropriate subjects, and such reference levels may be tailored to specific populations of subjects (e.g., a reference level may be age-matched or gender-matched so that comparisons may be made between marker levels in samples from subjects of a certain age or gender and reference levels for a sepsis condition state, phenotype, or lack thereof in a certain age or gender group).

According to a specific aspect, the methods described herein can be combined with any other diagnostic method for determining post-viral fatigue syndrome, specifically genetic risk factors or immunologic factors such as autoantibodies.

According to certain embodiments, the method described herein additionally comprises determining a level of at least one additional marker in a sample from said patient.

Specifically, fatty acid and/or oxylipin may be used for determining the level of additional markers in the methods and means of the invention.

Specifically, one or more or the following fatty acids and oxylipinsmay be determined in the methods of the invention as additional marker(s): DHA, 17- HDoHE, 22-HDoHE, polyunsaturated fatty acids, trans-isoforms of polyunsaturated acids, trans DHA, or 13-OxoODE.

Specifically, cytokines may be used as additional markers in the methods and means of the invention.

Specifically, one or more of the following cytokinesmay be determined in the methods of the invention as additional marker: IL-18, MCP-1, or TNF-RII, .

Specifically, SERPINA1 may be used as an additional marker.

In particular aspects, the methods described herein utilize more than one marker in a multimarker panel, which includes at least one of the specific markers described herein. Specifically, the multimarker panel is a post-viral fatigue syndrome multimarker panel which comprises or consists of those markers of relevance to said post-viral fatigue syndrome, in particular chronicpost-COVID-19 syndrome or long-COVID. Specifically, said multimarker panel comprises or consists of SERPINA5, and one or more of tryptophan-betaine, taurine, valine, biotinidase, and/or vitamin D binding protein. Specifically, the post-viral fatigue syndrome multimarker panel described herein may provide for an expression profile associated with said post-viral fatigue syndrome. Such profile may provide a highly sensitive and specific test with both high positive and negative predictive values permitting diagnosis and prediction of the patient's risk of developing disease or disease progression, in particular the risk of developing post-viral fatigue syndrome, more particular chronicpost-COVID-19 syndrome or long-COVID.

According to a specific embodiment, provided is a post-viral fatigue syndrome specific multimarker panel comprising or consisting of two, three, four, five, or six of the biomarkers SERPINA5, tryptophan-betaine, taurine, valine, biotinidase, and vitamin D binding protein.

According to a specific embodiment, the multimarker panel may comprise further biomarkers.

According to a specific embodiment, the multimarker panel comprises or consists of at least 2, 3, 4, 5, or 6 different markers. Specifically, the multimarker panel is composed of a number of different biomarkers up to 100, 90, 80, 70, 60, 50, 40, 30, 20, or 10, in particular up to 10, 9, 8, or 7.

The same or different types of markers may be determined when analysing a sample using the multimarker panel described herein. In specific embodiments, at least one marker of a multimarker panel is determined by a polypeptide or protein as expression product indicating said marker expression. In specific embodiments, at least one marker of a multimarker panel is determined by a metabolite or as production product of metabolism indicating said marker production. In further specific embodiments, each marker of a multimarker panel may be a polynucleotide, such as mRNA or transcripts, as expression product indicating said marker expression.

The multimarker panel can be placed on an array such as a microarray so that the expression status of each of the markers is assessed side-by-side or simultaneously.

The markers described herein are also referred to as "biomarkers", and a collection (e.g., a combination or array) of markers is also referred to as "panel". Specific markers described herein are particularly characterized by their encoded amino acid sequence, or the coding nucleotide sequence. Specific markers described herein are metabolites. Exemplary protein sequences to the recited markers are provided herein. Exemplary chemical structures of the recited markers are provided herein. Protein sequences may or may not comprise an N-terminal secretion signal sequence. Such signal sequence is typically not meant to be part of the marker sequence. It is understood that a marker is specifically characterized by a respective marker coding sequence (or gene), or sequences encoded by a respective marker gene, including naturally-occurring isomers.

The invention further provides for a set of reagents (a kit of parts) to determine the production of any of the markers described herein. The term "kit" is specifically understood as "diagnostic kit" and refers to a kit or set of parts, which in combination or mixture can be used to carry out the measurement/detection of one or more analytes or markers.

According to a specific embodiment, the set further comprises means to prepare a fraction of a blood sample. Such means include buffer or other auxiliary reagents or tools to enrich and/or fractionate white blood cells, cell lysis reagents, internal controls, negative controls, etc.

According to a specific aspect, a diagnostic kit is provided which comprises the multimarker panel described herein and means as necessary for determining the production of each marker of said multimarker panel. For example, ligands specifically recognizing production products of each marker of said multimarker panel may be present in the diagnostic kit. Preferably, the kit comprises a ligand being a specific binder for each of the markers. Specifically, the diagnostic kit is provided, which does not comprise any further marker panels and further specific ligands, other than said multimarker post-viral fatigue syndrome panel described herein and respective specific ligands.

Specifically, the kit described herein may contain at least a detection molecule and/or a binder (e.g. a primer, probe or an immunoagent), wherein the detection molecule and/or the binder specifically recognizes the marker or the respective analyte, or a reaction product of such marker or analyte. In addition, various reagents or tools may be included in the kit. The diagnostic kit preferably comprises all essential components to determine the amount of the marker in the biological sample, optionally without common or unspecific substances or components, such as water, buffer or excipients that may be conveniently added when performing the analysis. The diagnostic kit may comprise any useful reagents for carrying out the subject methods, including substrates or solid surfaces, such as microbeads or planar arrays or wells, reagents for marker isolation, detection molecules directed to specific targets, detectable labels, solvents, buffers, linkers, various assay components, blockers, and the like.

A kit, herein also referred to as "set", may also include instructions for use in a diagnostic method. Such instructions can be, for example, provided on a device included in the kit, e.g. tools or a device to prepare a biological sample for diagnostic purposes, such as separating a cell and/or protein containing fraction before determining a marker. The kit may conveniently be provided in storage stable form, such as a commercial kit with a shelf-life of at least 6 months. The storage stable kit can be stored preferably at least 6 months, more preferably at least 1 or 2 years. It may be composed of dry (e.g. lyophilized) components, and/or include preservatives.

The preferred diagnostic kit is provided as a packaged or prepackaged unit, e.g. wherein the components are contained in only one package, which facilitates routine experiments. Such package may include the reagents necessary for one or more tests, e.g., suitable to perform the tests of a series of biological samples. The kit may further suitably contain a marker preparation as a standard or reference control, which may be the specific marker described herein optionally comprising a label.

The preferred set further contains reagents for determining the expression of at least one of the markers further described herein, e.g., tryptophan-betaine, Taurine, valine, SERPINA5, Biotinidase and vitamin D binding protein, optionally together with further tools to perform such analysis.

According to a further specific aspect, a preferred set contains one or more ligands specifically recognizing a marker as a binding partner, and/or reagents specifically recognizing the product of binding the ligand to such marker.

The term "specifically recognizing" as used herein (also referred to as "specific binding") is meant that the binding partner or ligand reacts or associates more frequently, more rapidly, with greater duration and/or with greater affinity with a particular target biomarker than it does with alternative biomarkers.

Marker specific ligands are substances which can bind to or detect at least one of the markers for a detection method described herein and are in particular marker nucleotide sequence detecting tools or marker protein specific antibodies, including antigen-binding antibody fragments, such as Fab, F(ab), F(ab)', Fv, scFv, or single chain antibodies. Marker specific substances can also be selected from marker nucleotide sequence specific oligonucleotides, which specifically bind to a portion of the marker sequences, e.g. mRNA or cDNA, or are complementary to such a portion in the sense or complementary anti-sense orientation, like a cDNA complementary strand. The preferred ligands may be attached to solid surfaces, including beads, to catch and separate the marker.

According to specific embodiments, a ligand can be labelled. Biological assays require methods for detection, and one of the most common methods for quantitation of results is to conjugate a detectable label to a protein or nucleic acid that has affinity for one of the components in the biological system being studied. Detectable labels may include molecules that are themselves detectable (e.g., fluorescent moieties, electrochemical labels, metal chelates, etc.) as well as molecules that may be indirectly detected by production of a detectable reaction product (e.g., enzymes such as horseradish peroxidase, alkaline phosphatase, etc.) or by a specific binding molecule which itself may be detectable (e.g., biotin, digoxigenin, maltose, oligohistidine, 2,4-dintrobenzene, phenylarsenate, ssDNA, dsDNA, etc.).

Specific ligands are immunoagents, such as those particularly used in an analytical method.

The term "immunoagent" as used herein is understood as a target-specific molecule that contains a target binding site that specifically binds or immunoreacts with said target, such as a target antigen. Preferred immunoagents are antibodies or antigen-binding fragments thereof e.g., monoclonal or polyclonal antibodies or respective antibody fragments. Particularly, antibodies that are specifically binding to the biomarker are employed in an immunoassay for determining the biomarker.

Specific immunoagents may be capture molecules or molecular scaffolds, which are understood as molecules that may be used to bind target molecules or molecules of interest, i.e. analytes such as the marker described herein, from a sample. Such molecules are shaped adequately, both spatially and in terms of surface features, such as surface charge, hydrophobicity, hydrophilicity, presence or absence of Lewis donors and/or acceptors, to specifically bind the target molecule. Hereby, the binding may, for instance, be mediated by ionic, van-der-Waals, pi-pi, sigma-pi, hydrophobic or hydrogen bond interactions or a combination of two or more of the aforementioned interactions or covalent interactions between the capture molecules or molecular scaffold and the target molecule. Capture molecules or molecular scaffolds may for instance be selected from the group consisting of a nucleic acid molecule, a carbohydrate molecule, a PNA molecule, a protein, a peptide and a glycoprotein, for example, aptamers, DARpins^{®} (Designed Ankyrin Repeat Proteins), Affimers^{®} and the like.

Specifically, the immunoagent is considered to be specifically recognizing a target such as a marker described herein, if its affinity towards the target is at least 100-fold or 1000-fold higher than towards other molecules comprised in the sample containing the marker. It is well known in the art how to develop and to select antibodies with a given specificity to recognize a target antigen.

According to a specific aspect, the immunoassay may employ at least one antibody which is labeled, and another antibody that is bound to a solid phase or can be bound selectively to a solid phase. The first antibody and the second antibody can be present dispersed in a liquid reaction mixture, and a first labeling component may be bound to the first antibody, and a second labeling component of said labeling system may be bound to the second antibody so that, after binding of both antibodies to the biomarker to be detected, a measurable signal which permits detection of the resulting sandwich complexes in the measuring solution is generated.

According to a specific aspect, the method described herein may be performed as an immunoassay comprising the steps of:
a) contacting the sample with a first antibody or an antigen-binding fragment thereof, specific for a first epitope of the marker, and with second antibody or an antigen-binding fragment thereof, specific for a second epitope of the marker; and
b) detecting the binding of the first and second antibodies or antigen-binding fragments thereof to the marker.

In particular, the first antibody and the second antibody may be present dispersed in a liquid reaction mixture, and wherein a first labelling component is bound to the first antibody, and/or a second labelling component of said labelling system Is bound to the second antibody so that, after binding of both antibodies to at least one marker or fragment thereof, a measurable signal which permits detection of the resulting sandwich complexes in the measuring solution is generated.

In specific cases, the method is carried out as sandwich immunoassay, specifically wherein one of the antibodies is immobilized on a solid phase, for example, the walls of coated test tubes, microtiter plates, or magnetic particles, and another antibody comprises a detectable label or means enabling for selective attachment to a label, and which serves the detection of the formed sandwich structures.

The set may further suitably contain a preparation as a standard or reference control. Specifically, a negative control or reference control composition may be used. Specifically, a positive reference composition may be used. Specifically, said positive reference may be suitable marker molecules.

In such case, a detection signal may be normalized to a control signal corresponding to the amount of signal produced by the control composition, thereby detecting the presence or level of the protein determined in the sample. Normalizing the detection signal may comprise subtracting the control signal from the detection signal.

The set may be particularly used in a method described herein.

The determination of high or low levels of the markers described herein has turned out to be highly reliable with respect to determining a post-viral fatigue syndrome, differentiating said post-viral fatigue syndrome from other types of fatigue syndrome such as non-viral fatigue syndrome or metabolic fatigue syndrome, or post-viral fatigue syndrome progression, such that it enables an appropriate action by a medical professional.

### EXAMPLES

The examples described herein are illustrative of the present invention and are not intended to be limitations thereon. Many modifications and variations may be made to the techniques described and illustrated herein without departing from scope of the invention. Accordingly, it should be understood that the examples are illustrative only and are not limiting the scope of the invention.

### Example 1

An exploratory study was set up to perform mass spectrometry based proteomics, metabolomics and lipidomics analyses from blood specimen from LCS patients and compared them to specimen from COVID-19 patients who had fully recovered (termed post-COVID/healthy) and healthy donors after vaccination (termed healthy vaccinated). Using this approach enabled to identify alterations in all three categories of molecules, which are apparently interlinked with regard to the underlying pathophysiology. Indeed, it was enabled to make rational suggestions in that regard, identifying macrophages and related cells as key players and disturbed mitochondrial metabolism as key feature of long covid and chronic fatigue syndrome.

### Methods

### Patient recruitment and sample acquisition

The age and gender matched recovered/healthy and the healthy/vaccinated study groups were recruited among volunteers after calls at the Vienna General Hospital/Medical University of Vienna and the University of Applied Sciences, Vienna, Austria. All vaccinated participants had received two doses of either the vector-based vaccine Vaxzevria (Astra Zeneca, Oxford, UK) or the mRNA-based vaccine Comirnaty (Pfizer, New York City, NY, USA). Serum and EDTA-anticoagulated plasma were obtained by peripheral venous blood draw. Serum samples were left for 15 min to allow for clotting before centrifugation for 15 min at 1500 g at 4 °C while EDTA plasma samples were immediately centrifuged after blood collection. Following these steps, all samples were immediately frozen at -80 °C until analyses.

### Determination of anti SARS-CoV-2 antibody status

Antibody levels against SARS-CoV-2 Spike protein (anti-S) and Nucleocapsid Protein (anti-N) were determined from sera of the study participants using the Elecsys Anti-SARS-CoV-2 S immunoassay as well as the qualitative Elecsys Anti-SARS-CoV-2 immunoassay (detecting SARS-CoV-2 N protein) on a cobas e801 analyzer (Roche Diagnostics, Rotkreuz, Switzerland). Analyses were performed at the diagnostic laboratory at the Department of Laboratory Medicine, Medical University of Vienna (certified acc. to ISO 9001:2015 and accredited acc. to ISO 15189:2012).

### Determination of routine laboratory parameters

Total protein, albumin, ferritin, CRP, HDL, LDL, LP(a) were determined using standard routine diagnostic tests on a cobas e801 analyzer (Roche) at the diagnostic laboratory at the Department of Laboratory Medicine, Medical University of Vienna.

### Determination of serum cytokines

Undiluted blood serum samples were analyzed using the ProcartaPlex^{™} Multiplex Immunoassay (Human immune monitoring 65 Plex, Thermo Fisher Scientific, Reference Number MAN0017980) according to manufacturer's instruction. Of the 65 analytes, 51 were below the lower limit of quantification in >95% of all samples (irrespective of group) and were therefore excluded from further analysis. Measurements and analysis of all Human ProcartaPlex Immunoassays were performed on a Luminex 200 instrument (Luminex Corp., Austin, Tx, USA) as described in detail before (J. J. Kovarik *et al.,* 2021).

### Plasma proteomics

For untargeted plasma proteomics analyses, EDTA-anticoagulated plasma samples were diluted 1:20 in lysis buffer (8 M urea, 50 mM TEAB, 5 % SDS), heated at 95 °C for 5 min prior the protein concentration was determined using a BCA assay. For enzymatic protein digestion, 20 µg of protein was used and the ProtiFi S-trap technology (A. Zougman, et al 2014) applied. Briefly, solubilized protein was reduced and carbamidomethylated by adding 64 mM dithiothreitol (DTT) and 48 mM iodoacetamide (IAA), respectively. Prior to sample loading onto the S-trap mini cartridges, trapping buffer (90 % v/v methanol, 0.1M triethylammonium bicarbonate) was added. Thereafter, samples were thoroughly washed and subsequently digested using Trypsin/Lys-C Mix at 37 °C for two hours. Finally, peptides were eluted, dried and stored at -20 °C until LC-MS analyses.

LC-MS/MS analysis was performed as described previously (Seiser S. *et al.,* 2021). In short, Reconstitution of dried peptide samples was achieved by adding 5 µL of 30 % formic acid (FA) containing 4 synthetic standard peptides and subsequent dilution with 40 µL of loading solvent (97.9 % H2O, 2 % ACN, 0.05 % trifluoroacetic acid). Thereof, 1 µL were injected into the Dionex Ultimate 3000 nano high performance liquid chromatography (HPLC)-system (Thermo Fisher Scientific). In order to pre-concentrate peptides prior to chromatographic separation, a pre-column (2 cm × 75 µm C18 Pepmap100; Thermo Fisher Scientific) run at a flow rate of 10 µL/min using mobile phase A (99.9 % H2O, 0.1 % FA) was used. The subsequent peptide separation was achieved on an analytical column (25 cm × 75 µm 25 cm Aurora Series emitter column (lonopticks)) by applying a flow rate of 300 nL/min and using a gradient of 7 % to 40 % mobile phase B (79.9 % ACN, 20 % H2O, 0.1 % FA) over 43 min, resulting in a total LC run time of 85 min including washing and equilibration steps. Mass spectrometric analyses were performed using the timsTOF Pro mass spectrometer (Bruker) equipped with a captive spray ion source run at 1650 V. Further, the timsTOF Pro mass spectrometer was operated in the Parallel Accumulation-Serial Fragmentation (PASEF) mode and a moderate MS data reduction was applied. A scan range (m/z) from 100 to 1700 to record MS and MS/MS spectra and a 1/k0 scan range from 0.60 - 1.60 V.s/cm2 resulting in a ramp time of 100 ms to achieve trapped ion mobility separation were set as further parameters. All experiments were performed with 10 PASEF MS/MS scans per cycle leading to a total cycle time of 1.16 s. Furthermore, the collision energy was ramped as a function of increasing ion mobility from 20 to 59 eV and the quadrupole isolation width was set to 2 Th for m/z < 700 and 3 Th for m/z > 700.

Subsequent LC-MS data analysis was performed using the publicly available software package MaxQuant 1.6.17.0 running the Andromeda search engine (Cox J.& Mann M., 2008). Protein identification as well as label-free quantification (LFQ) was achieved by searching the raw data against the SwissProt database "homo sapiens" (version 141219 with 20380 entries). General search parameter included an allowed peptide tolerance of 20 ppm, a maximum of 2 missed cleavages, carbamidomethylation on cysteins as fixed modification as well as methionine oxidation and N-terminal protein acetylation as variable modification. A minimum of one unique peptide per protein was set as search criterium for positive identifications. In addition, the "match between runs" option was applied, using a 0.7 min match time window and a match ion mobility window of 0.05 as well as a 20 min alignment time window and an alignment ion mobility of 1. An FDR≤0.01 was set for all peptide and protein identification.

LC-MS data evaluation as well as statistical analysis was accomplished using the Perseus software (version 1.6.14.0) (Cox J., Mann M., 2012). First, identified proteins were filtered for reversed sequences as well as common contaminants and annotated according to the different study groups. Prior to statistical analysis, LFQ intensity values were transformed (log2(x)), and proteins were additionally filtered for their number of independent identifications (a minimum of 5 identifications in at least one group). Afterwards, missing values were replaced from a normal distribution (width: 0,3; down shift: 1,8). Two-sided t-tests as well as statistics for volcano plots were performed applying an FDR of 0.05 and a SO of 0.1, whereby SO controls the relative importance of t-test p-value and difference between the means. Histograms were generated using GraphPad Prism Version 6.07 (2015).

### Plasma lipidomics

The analysis of fatty acids and oxylipins was performed essentially as described (Niederstaetter L. *et al.,* 2021). Frozen EDTA-anticoagulated plasma was freshly thawed on ice. For precipitation of proteins, plasma (300 µL) was mixed with cold EtOH (1.2 mL, abs. 99%, -20°C; AustroAlco) including an internal standard mixture of 12S-HETE-d8, 15S-HETE-d8, 5-Oxo-ETE-d7, 11, 12-DiHETrE-d11, PGE2-d4 and 20-HETE-d6 (each 100 nM; Cayman Europe, Tallinn, Estonia). The samples were stored over-night at - 20°C. After centrifugation (30 min, 5000 rpm, 4°C), the supernatant was transferred into a new 15 mL Falcon^{™} tube. EtOH was evaporated *via* vacuum centrifugation at 37°C until the original sample volume (300 µl) was restored. For solid phase extraction (SPE) samples were loaded onto preconditioned StrataX SPE columns (30 mg mL-1; Phenomenex, Torrance, CA, USA) using Pasteur pipettes. After sample loading, the SPE columns were washed with 5 mL of MS grade water and eluted with ice-cold MeOH (500 µL; MeOH abs.; VWR International, Vienna, Austria) containing 2% formic acid (FA; Sigma-Aldrich). MeOH was evaporated using a gentle nitrogen stream at room temperature and the dried samples were reconstituted in 150 µL reconstitution buffer (H₂O:ACN:MeOH + 0.2% FA-vol% 65:31.5:3.5) containing an internal standard mixture of 5S-HETE-d8, 14,15-DiHETrE-d11 and 8-iso-PGF2a-d4 (10-100 nM; Cayman Europe, Tallinn, Estonia). The samples were then transferred into an autosampler held at stored at 4°C and subsequently measured via LC-MS/MS.

For LC-MS analyses, analytes were separated using a Thermo Scientific^{™} Vanquish^{™} (UHPLC) system equipped with a Kinetex^{®} C18-column (2.6 µm C18 100 Å, LC Column 150 × 2.1 mm; Phenomenex^{®}) applying a gradient flow profile (mobile phase A: H₂O + 0.2% FA, mobile phase B: ACN:MeOH (vol% 90:10) + 0.2% FA) starting at 35% B and increasing to 90% B (1-10 min), further increasing to 99% B within 0.5 min and held for 5 min. Solvent B was then decreased to the initial level of 35% within 0.5 min and the column was equilibrated for 4 min, resulting in a total run time of 20 min. The flow rate was kept at 200 µL min-1 and the column oven temperature at 40°C. The injection volume was 20 µL and all samples were analysed in technical duplicates. The Vanquish UHPLC system was coupled to a Q Exactive^{™} HF Quadrupole-Orbitrap^{™} high-resolution mass spectrometer (Thermo Fisher Scientific, Austria), equipped with a HESI source for negative ionization to perform the mass spectrometric analysis. The MS scan range was 250-700 m/z with a resolution of 60,000 (at m/z 200) on the MS1 level. A Top 2 method was applied for fragmentation (HCD 24 normalized collision energy), preferable 33 m/z values specific for well-known eicosanoids and precursor molecules from an inclusion list. The resulting fragments were analysed on the MS2 level at a resolution of 15,000 (at m/z 200). Operating in negative ionization mode, a spray voltage of 2.2 kV and a capillary temperature of 253 °C were applied. Sheath gas was set to 46 and the auxiliary gas to 10 (arbitrary units).

For subsequent data analysis, raw files generated by the Q Exactive^{™} HF Quadrupole-Orbitrap^{™} high-resolution mass spectrometer were checked manually via Thermo Xcalibur^{™} 4.1.31.9 (Qual browser) and compared with reference spectra from the Lipid Maps depository library from July 2018 (E. Fahy E. et al., 2007). Peak integration was performed using the TraceFinder^{™} software package (version 4.1-Thermo Scientific, Vienna, Austria). Principal Component Analysis and volcano plots were generated using the Perseus software (version 1.6.14.0) applying an FDR of 0.05 (Cox J., Mann M., 2012). Therefore, data were normalised to the internal standards and missing values were replaced by a constant which corresponds to the half of the lowest normalized area under the curve (nAUC) value of each individual eicosanoid. Histograms were generated using GraphPad Prism Version 6.07 (2015). The ratio of Ω-3/Ω6 fatty acids depicted in Figure 2 reflects the ratio of the omega-3 polyunsaturated fatty acids eicosapentaenoic acid (EPA) and docosahexaenoic (DHA) to the omega-6 polyunsaturated fatty acids arachidonic acid (AA) and dihomo-gamma-linolenic acid (DGLA).

### Plasma metabolomics

EDTA plasma samples (10 µL) of study participants were analyzed by a targeted metabolomic assay. Targeted metabolomics experiments were conducted using the MxP^{®} Quant 500 Kit (Biocrates Life Sciences AG, Innsbruck, Austria). We detected 630 analytes, including 40 acylcarnithines, 1 alkaloid, 1 amine oxide, 50 amino acid related metabolite, 14 bile acids, 9 biogenic amines, 7 carboxylic acids, 28 ceramides, 22 cholesteryl esters, 1 cresol, 44 diacylglycerols, 8 dihydroceramides, 12 fatty acids, 90 glycerophospholipids, 34 glycosylceramides, 4 hormones, 4 indole derivatives, 2 nucleobase related metabolites, 15 sphingolipids, 242 triacylglycerols, the sum of hexoses and 1 vitamin/cofactor. A total of 474 metabolites showed signal intensities within the quantification window and were further evaluated. Measurements were carried out using LC-MS and flow injection (FIA)-MS analyses on a Sciex 6500+ series mass spectrometer coupled to an ExionLC AD chromatography system (AB Sciex, Framingham, MA, USA), utilizing the Analyst 1.7.1 software with hotfix 1 (also AB SCIEX). All required standards, quality controls and eluents were included in the kit, as well as the chromatographic column for the LC-MS/MS analysis part. Phenyl isothiocyanate (Sigma-Aldrich, St. Louis, USA) was purchased separately and was used for derivatization of amino acids and biogenic amines according to the kit manual. Preparation of the measurement worklist as well as data validation and evaluation was performed with the software supplied with the kit (MetIDQ-Oxygen-DB110-3005, Biocrates Life Sciences). Principal Component Analysis and volcano plots were generated using the Perseus software (version 1.6.14.0) applying an FDR of 0.05 (Cox J., Mann M., 2012). Histograms were generated using GraphPad Prism Version 6.07 (2015). The heatmap including 126 triacylglycerols was generated by dividing the concentration of each lipid through the average concentration of this lipid over all samples using Excel.

### Results

### Patient characteristics and study design

Serum and plasma samples of LCS patients were obtained from the Division of Cardiology of the Department for Internal Medicine II of the Medical University of Vienna. All patients had succumbed to PCR positive SARS-CoV-2 infection at least three months before presentation. Post infection status was confirmed by positive antibody testing for anti-N(ucleocapsid) and anti-S(pike) antibodies. All patients had a positive anamnesis of chronic fatigue and/or severe chronic lack of concentrations following SARS-CoV-2 infection combined with at least one more chronic symptom including dyspnea, coughing and loss of smelling among others, qualifying them for the diagnosis LCS (whole patient characteristics are displayed in Table 1A and 1B). In parallel, a cohort of age- and sex-matched individuals was recruited who had a similar SARS-CoV-2 infection history but were symptom free in anamnesis at the time of blood draw (termed recovered; R). Again, infection status was confirmed by positive anti-N and anti-S antibody levels. Similarly, a third cohort of healthy individuals with no history of SARS-CoV-2 infection was recruited three months after full anti-SARS-CoV-2 immunization which was also confirmed by anti-N (-)/anti-S (+) status (termed healthy; H). For the here described exploratory study, specimen from 13 individuals from each group were selected for broad scale multi-omics analyses including a cytokine array, untargeted shotgun proteomics, a comprehensive commercial metabolomics kit and an untargeted eicosanoid/docosanoid assay. Routine laboratory testing of basic protein, lipid and lipoprotein parameters showed no differences between the three groups. Similarly, CRP levels in all three groups were below or only minimally above the threshold (Table 2). Furthermore, none of the analyzed individuals presented with fever or displayed any clinical signs of systemic infection at the time of blood withdrawal. Taken together these results rule out major systemic metabolic or acute inflammatory processes in the tested individuals at time of blood draw.

To confirm the hypothesis regarding the role of fatty acids in long-covid and to exclude an effect from oral supplementation, EDTA-plasma samples from a fourth cohort including 10 healthy subjects have been analyzed which have not been exposed to COVID-19. For this purpose, these subjects received tablets containing 870mg Omega-3 (420 mg EPA and 330 mg DHA) twice daily in a prospective study design for one week and plasma samples were obtained before start of intake and after intake of the last dose.

**Table 1: Study group characteristics**

| **Table 1A** | **healthy (n=13)** | **recovered (n=13)** | **long-covid (n=13)** |
|---|---|---|---|
| General data | | | |
| Gender (f:m) | 7:6 | 7:6 | 9:4 |
| Age [years] | 30 (25-43) | 32 (24-49) | 33 (21-53) |
| Time after exposure [months] | 6 (3-8) | 10 (3-12) | 7 (3-10) |

| **Chronic disease** (total number (percentage)) | | | |
|---|---|---|---|
| Asthma bronchiale | 0 | 1 (0.07) | 4 (0.31) |
| Multiple sclerosis | 0 | 0 | 1 (0.07) |
| Autoimmune thyreoiditis | 0 | 1 (0.07) | 1 (0.07) |
| Atopic dermatitis | 0 | 1 (0.07) | 0 |
| Psoriasis arthritis | 1 (0.07) | 0 | 0 |

| **Medication** (total number (percentage)) | | | |
|---|---|---|---|
| SSRI | 0 | 0 | 2 (0.15) |
| Inhalative glucocorticoids | 0 | 1 (0.07) | 5 (0.38) |
| Systemic antihistamines | 0 | 0 | 2 (0.15) |
| LTA | 0 | 0 | 1 (0.07) |
| PEG interferon | 0 | 0 | 1 (0.07) |
| Levothyroxin | 0 | 1 (0.07) | 1 (0.07) |
| Sulfasalazine | 1 (0.07) | 0 | 0 |
| ACE inhibitors | 0 | 1 (0.07) | 0 |

| **Table 1B** | **healthy (n=13)** | **recovered (n=13)** | **long-covid (n=13)** |
|---|---|---|---|
| **Chronic Symptom** (total number (percentage)) | | | |
| Lack of concentration | - | - | 10 (0.77) |
| Fatigue | - | - | 9 (0.69) |
| Dyspnoea | - | - | 9 (0.69) |
| Chronic cough | - | - | 2 (0.15) |
| Muscular aching | - | - | 3 (0.23) |
| Amnesic aphasia | - | - | 5 (0.38) |
| Sleep disorder | - | - | 4 (0.31) |
| Urinary incontinence | - | - | 2 (0.15) |
| Nausea | - | - | 3 (0.23) |
| Tinnitus | - | - | 4 (0.30) |
| Other symptoms | - | - | 9 (0.69) |

**Table 2: Routine serum parameters**

| | **healthy (n=13)** | **recovered (n=13)** | **long-covid (n=13)** |
|---|---|---|---|
| total protein [g/L] | 72.7 [65.2-77.5] | 73.10 [67.5-78.7] | 75.3 [63.5-84.3] |
| albumin [g/L] | 49.7 [46.3-54.4] | 48.5 [41-53.4] | 50.7 [43.7-55.6] |
| triglyceride [mg/dL] | 58 [36-166] | 115 [49-353] | 77 [48-159] |
| cholesterol [mg/dL] | 171 [108-198] | 207 [146-281] | 176 [100-192] |
| HDL [mg/dL] | 60 [36-94] | 61 [49-93] | 54 [37-74] |
| LDL [mg/dL] | 85.2 [53.6-129.2] | 102.6 [76.2-201.2] | 98.2 [38.6-122.8] |
| LP(a) [nmol/L] | 18 [0-235] | 13 [0-421] | 15 [0-135] |
| CRP [mg/L] | 0.4 [0-5.7] | 1 [0-3] | 0.6 [0-6.2] |
| ferritin [µg/L] | 51 [15.1-175.1] | 91 [22.4-565] | 52.6 [18.7-207.6] |

### Cytokine analysis indicates a lack of pro-inflammatory processes in long-COVID patients.

Cytokines regulate inflammatory mechanisms including cell communication of immune cells as required to cope with infectious pathogens. In order to investigate whether long-COVID may result from still unresolved inflammation after the viral infection, a panel of 65 cytokines was assayed. Remarkably, neither pro- nor anti-inflammatory cytokines were found significantly up-regulated in long-COVID patients. Striking was the apparent down-regulation of IL-18 in long-COVID patients, as this T-cell derived cytokine is orchestrating antiviral response of macrophages (Figure 1A). Furthermore, the macrophage-derived cytokines MCP-1 and TNF-RII were found significantly down-regulated in long-COVID patients compared to fully recovered patients (Figure 1A). Generally, cytokine levels were rather low in post-COVID patients, indicating a lack of pro-inflammatory activities.

### Proteome profiling corroborates a lack of inflammation in long-COVID patients and points at altered macrophage activities.

Shotgun proteomics identified 278 proteins after filtering for high confidence (FDR<0.01) and robustness (at least five independent identifications per protein per patient group). PCA of label-free shotgun proteomics of plasma samples separated the three patient groups in a distinct fashion. Vaccinated individuals were mainly distributed between the almost completely separated groups of long-COVID and fully recovered patients (Figure 1B). Thus, significant (FDR < 0.05) proteome alterations were mainly observed comparing long-COVID to post-COVID patients (Figure 1C). Beside several cell leakage products typically derived from uncontrolled cell death, including actin (ACT), tubulin (TUBA1B), myosin-9 (MYH9) and profilin (PFN1), only SERPINA1 and vitamin D binding protein (GC), were found significantly up-regulated. A larger number of proteins was found down-regulated, including the protease inhibitors SERPINA5 and SERPINF2 and the macrophage-associated proteins CD14, ANG and PRG4. SERPINA5, biotinidase, and Vitamin D-binding protein are advantageous due to the relative high contrasts between healthy and diseased individuals, robustness of measurement and low susceptibility to artifacts introduced by the sampling method and analysis procedure. The acute phase protein CRP was only detected in a few samples and thus removed upon filtering. Other acute phase proteins including serum amyloid A and P component, fibrinogens, orosomucoid and alpha-2-macroglobulin were readily detectable but rather down-regulated in long-COVID patients. This finding strongly supports the absence of a pro-inflammatory phenotype detected in long-COVID patients. The proteins SERPINA5, biotinidase, and Vitamin D-binding protein are significantly deregulated (Figure 1D) and directly relate to long-COVID pathomechanisms. The data for SERPINA5, biotinidase, and Vitamin D-binding protein are shown in Table 3.

### Fatty acid and oxylipin analysis indicate increased phospholipase A2 activities affecting preferentially mitochondrial lipids

Polyunsaturated fatty acids such as arachidonic acid and oxidized products such as eicosanoids represent highly bioactive but short-lived signaling molecules and lipid mediators modulating inflammation and other immune functions (Dennis, E. A., & Norris, P. C., 2015). Typically, inflammation-associated activation of phospholipase A2 releases unsaturated fatty acids from the sn2 position of phospholipids. The bioactive oxylipins are subsequently formed from these fatty acids by the action of cyclooxygenases, lipoxygenases and cytochrome P450 monooxygenases. PCA analysis of fatty acids and their oxidation products rather separated healthy controls from long-COVID patients, with the group of recovered patients dispersed in between (Figure 2A). Thus, here most of the significant events were observed comparing long-COVID patients to healthy controls (Figure 2B). Evidently, higher levels of all kinds of fatty acids were characteristic for long-COVID, pointing to higher phospholipase activities. However, the predominant release of DHA into the blood of long-COVID patients (Figure 2B, C) deserves attention. Chronic inflammation caused e.g. by atherosclerotic cardiovascular diseases are typically associated with increased levels of arachidonic acid (Zhang, Tet al. 2021), as presently observed in case of recovered patients (Figure 2C). Blood plasma levels of fatty acids may be affected by confounders such as nutrition. However, the presently observed significant long-COVID-associated increase in DHA is corroborated by two independent observations. First, the DHA oxidation products 17- and 22-HDoHE were also found significantly increased in long-COVID patients (Figure 2D). Second, polyunsaturated fatty acids may occur together with their trans-isoforms. It is observable that nutritional supplementation of DHA decreases relative concentrations of trans-DHA (Figure 2E). In contrast, the levels of trans-DHA were consistently higher in long-COVID patients, suggesting that DHA was released from intracellular sources potentially exposed to radical stress. The ratio of Q3-fatty acids to Ω6-fatty acids was found significantly increased in long-COVID patients (Figure 2F). Remarkably, the anti-inflammatory 15-LOX product from linoleic acid, 13-OxoODE, was also found increased in long-COVID patients when compared to recovered patients (Figure 2E). Thus, here it is suggested that the formation of anti-inflammatory oxylipins may be characteristic for long-COVID patients.

### Metabolomic aberrations relate to chronic fatigue syndrome and display an osmolyte-mediated anti-inflammatory signature

In case of metabolites, PCA analysis indicated maximal contrast between recovered and long-COVID patients (Figure 3A) as observed in case of plasma proteins. Indeed, out of 580 metabolites and lipids analyzed using the Biocrates MxP Quant 500 kit, 474 were successfully quantified and 107 were found significantly deregulated between these two groups (Figure 3B). Most apparently, a large number of metabolic alterations were related to a disturbance in energy metabolism, as evidenced by the down-regulation of branched chain amino acids Val, Leu and lie, the aromatic amino acid Tyr and the inflammation modulators Trp and Arg (Figure 3C). The up-regulation of C18:1-acylcarnitine accompanied with a down-regulation of C3-acylcarnitine may indicate reduced levels of beta oxidation accompanied by increased levels of amino acid catabolism. A further indication for a disturbed energy metabolism was the significant increase in lactate, pointing to increased levels of glycolysis. Intriguingly, the majority of lipids including various triacylglycerols, glycosylceramides, glycerophospholipids and several ceramides were found down-regulated in long-COVID (Figure 3D). A long-COVID-associated prevalence for anti-inflammation was evidenced by the present metabolomics results. The metabolite most significantly up-regulated in long covid-patients was tryptophan-betaine, also known as hypaphorine, an anti-inflammatory alkaloid (Sun, H. et al., 2017) Tryptophan-betaine may act as osmolyte in way similar to the anti-inflammatory molecule taurine (Figure 3C), which was also significantly up-regulated in long-COVID. Hypoxanthine, a marker for hypoxia, was found significantly up-regulated in recovered patients. This finding, in line with the herein described cytokine and proteome profiles, suggests that symptom-free post-COVID patients have not yet fully resolved virus-related disease mechanisms including hypoxia, but rather show successful management of virus-related inflammation and cell stress.

The data for valine, tryptophan-betaine, and taurine are shown in Table 3.

**Table 3**

| | **Tryptophan-betaine** | **Taurine** | **Valine** | **SERPINA5** | **Biotinidase** | **Vitamin D binding protein** |
|---|---|---|---|---|---|---|
| Healthy | 1.3 µM +/- 1.0 µM | 47 µM +/- 15 µM | 225 µM +/- 37 µM | 59 LFQ +/-15 LFQ | 60 LFQ +/- 31 LFQ | 851 LFQ +/- 128 LFQ |
| Recovered | 0.43 µM +/- 0.24µM) | 47 µM +/- 5µM | 230 µM +/- 42 µM | 71 LFQ +/- 24 LFQ | 92 LFQ +/- 32 LFQ | 658 LFQ +/- 90 LFQ |
| Long-COVID | 2.9 µM +/- 2.4 µM | 70 µM +/- 33 µM | 195 µM +/- 25 µM | 32 LFQ +/- 8 LFQ | 40 LFQ +/- 7 LFQ | 942 LFQ +/- 165 LFQ |

### REFERENCES

Bornstein, S. R., Voit-Bak, K., Donate, T., Rodionov, R. N., Gainetdinov, R. R., Tselmin, S., Kanczkowski, W., Müller, G. M., Achleitner, M., Wang, J., Licinio, J., Bauer, M., Young, A. H., Thuret, S., Bechmann, N., & Straube, R. (2022). Chronic post-COVID-19 syndrome and chronic fatigue syndrome: Is there a role for extracorporeal apheresis?. Molecular psychiatry, 27(1), 34-37. https://doi.org/10.1038/s41380-021-01148-4
Cox, J. & Mann, M. MaxQuant enables high peptide identification rates, individualized p.p.b.-range mass accuracies and proteome-wide protein quantification. Nat Biotechnol 26, 1367-1372, doi:10.1038/nbt.1511 (2008).
Cox J., Mann M., 1D and 2D annotation enrichment: a statistical method integrating quantitative proteomics with complementary high-throughput data. BMC Bioinformatics 13 Suppl 16, S12 (2012)
Dennis, E. A., & Norris, P. C. (2015). Eicosanoid storm in infection and inflammation. Nature reviews. Immunology, 15(8), 511-523. https:Hdoi.org/10.1038/nri3859
Fahy E., Sud M., Cotter D., Subramaniam S., LIPID MAPS online tools for lipid research. Nucleic Acids Res 35, W606-612 (2007)
Kovarik J. J. et al., Identification of Immune Activation Markers in the Early Onset of COVID-19 Infection. Front Cell Infect Microbiol 11, 651484 (2021)
Niederstaetter L. et al., Eicosanoid Content in Fetal Calf Serum Accounts for Reproducibility Challenges in Cell Culture. Biomolecules 11, (2021)
Overmyer, K. A., Shishkova, E., Miller, I. J., Balnis, J., Bernstein, M. N., Peters-Clarke, T. M., Meyer, J. G., Quan, Q., Muehlbauer, L. K., Trujillo, E. A., He, Y., Chopra, A., Chieng, H. C., Tiwari, A., Judson, M. A., Paulson, B., Brademan, D. R., Zhu, Y., Serrano, L. R., Linke, V., ... Jaitovich, A. (2021). Large-Scale Multi-omic Analysis of COVID-19 Severity. Cell systems, 12(1), 23-40.e7. https://doi.org/10.1016/j.cels.2020.10.003
Seiser S. et al., Octenidine-based hydrogel shows anti-inflammatory and protease-inhibitory capacities in wounded human skin. Sci Rep 11, 32 (2021)
Sun, H., Zhu, X., Cai, W., & Qiu, L. (2017). Hypaphorine Attenuates Lipopolysaccharide-Induced Endothelial Inflammation via Regulation of TLR4 and PPAR-γ Dependent on PI3K/Akt/mTOR Signal Pathway. International journal of molecular sciences, 18(4), 844. https://doi.org/10.3390/ijms18040844
Vijayakumar, B., Boustani, K., Ogger, P. P., Papadaki, A., Tonkin, J., Orton, C. M., Ghai, P., Suveizdyte, K., Hewitt, R. J., Desai, S. R., Devaraj, A., Snelgrove, R. J., Molyneaux, P. L., Garner, J. L., Peters, J. E., Shah, P. L., Lloyd, C. M., & Harker, J. A. (2022). Immuno-proteomic profiling reveals aberrant immune cell regulation in the airways of individuals with ongoing post-COVID-19 respiratory disease. Immunity, 55(3), 542-556.e5. https://doi.org/10.1016/j.immuni.2022.01.017
Weiss, T. et al. Schwann cell plasticity regulates neuroblastic tumor cell differentiation via epidermal growth factor-like protein 8. Nat Commun 12, 1624, doi:10.1038/s41467-021-21859-0 (2021).
Zougman A., Selby P. J., Banks R. E., Suspension trapping (STrap) sample preparation method for bottom-up proteomics analysis. Proteomics 14, 1006-1000 (2014)
Zhang, T., Zhao, J. V., & Schooling, C. M. (2021). The associations of plasma phospholipid arachidonic acid with cardiovascular diseases: A Mendelian randomization study. EBioMedicine, 63, 103189. https://doi.org/10.1016/j.ebiom.2020.103189

## Claims

1. A method for predicting post-viral fatigue syndrome in a human subject comprising determining the production of serine protease inhibitor SERPINA5 in a blood sample of said subject, wherein a reduced production of SERPINA5 is indicative of post-viral fatigue syndrome.

2. The method of claim 1, wherein the post-viral fatigue syndrome is chronic post-COVID-19 syndrome or long-COVID.

3. The method of claim 1 or 2, wherein the reduced production of SERPINA5 is indicative of post-viral fatigue syndrome if the production of SERPINA5 is reduced by 30 % compared to a predetermined reference value.

4. The method of any one of claims 1 to 3, wherein the blood sample is whole blood, serum, plasma, or a fraction of any of the foregoing.

5. The method of any one of claims 1 to 4, further comprising determining the production of at least one marker selected from tryptophan-betaine, taurine, valine, biotinidase, and vitamin D binding protein, preferably wherein:
a. increased production of tryptophan-betaine;
b. increased production of taurine;
c. reduced production of valine;
d. reduced production of biotinidase; and/or
e. increased production of vitamin D binding protein
are indicative of post-viral fatigue syndrome.

6. The method of any one of claims 1 to 5, wherein production of SERPINA5, tryptophan-betaine, taurine, valine, biotinidase, and vitamin D binding protein is determined, and
a. reduced production of SERPINA5;
b. increased production of tryptophan-betaine;
c. increased production of taurine;
d. reduced production of valine;
e. reduced production of biotinidase; and
f. increased production of vitamin D binding protein
are indicative of post-viral fatigue syndrome.

7. The method of any one of claims 1 to 6, wherein production is determined by measuring protein production, RNA production, or metabolite production, preferably quantitatively or semi-quantitatively, more preferably by immunoassays, targeted or untargeted assays based on mass spectrometry hyphenated with chromatography.

8. The method of any one of claims 1 to 7, wherein the subject receives a standard treatment of post-viral fatigue syndrome, chronic fatigue syndrome, chronic post-COVID-19 syndrome, or long-COVID.

9. The method of any one of claims 1 to 8, wherein the subject is monitored by determining and comparing the production of the marker(s) at different time points, preferably wherein the blood samples are drawn at least twice, at two or more different time points.

10. A method for diagnosing post-viral fatigue syndrome in a human subject comprising determining the production of SERPINA5 in a blood sample of said subject, wherein a reduced production of SERPINA5 is indicative of post-viral fatigue syndrome.

11. The method of claim 10, **characterized by** one or more of the following features:
a. the post-viral fatigue syndrome is chronic post-COVID-19 syndrome or long-COVID;
b. the reduced production of SERPINA5 is indicative of post-viral fatigue syndrome if the production of SERPINA5 is reduced by 30 % compared to a predetermined reference value;
c. the blood sample is whole blood, serum, plasma, or a fraction of any of the foregoing;
d. the production of at least one further marker selected from tryptophan-betaine, taurine, valine, biotinidase, and vitamin D binding protein is determined, preferably wherein
i. increased production of tryptophan-betaine;
ii. increased production of taurine;
iii. reduced production of valine;
iv. reduced production of biotinidase; and/or
v. increased production of vitamin D binding protein
are indicative of post-viral fatigue syndrome;
e. the production of SERPINA5, tryptophan-betaine, taurine, valine, biotinidase, and vitamin D binding protein is determined, and
i. reduced production of SERPINA5;
ii. increased production of tryptophan-betaine;
iii. increased production of taurine;
iv. reduced production of valine;
v. reduced production of biotinidase; and
vi. increased production of vitamin D binding protein
are indicative of post-viral fatigue syndrome;
f. the production is determined by measuring protein production, produced RNA, or produced metabolite, preferably quantitatively or semi-quantitatively, more preferably by immunoassays, targeted or untargeted assays based on mass spectrometry hyphenated with chromatography;
g. the subject receives a standard treatment of post-viral fatigue syndrome, chronic fatigue syndrome, chronic post-COVID-19 syndrome, or long-COVID;
h. the subject is monitored by determining and comparing the production of the marker(s) at different time points, preferably wherein the blood samples are drawn at least twice, at two or more different time points.

12. A method for monitoring treatment of a post-viral fatigue syndrome in a patient comprising determining the production of SERPINA5 in a blood sample of said patient, wherein increase of the production of SERPINA5 is indicative of the patient's response to said treatment.

13. The method of claim 12, which is **characterized by** one or more of the following features:
a. the post-viral fatigue syndrome is chronic post-COVID-19 syndrome or long-COVID;
b. the increased production of SERPINA5 is indicative of the patient's response to said treatment if the production of SERPINA5 is increased by 20 % compared to a reference sample;
c. the blood sample is whole blood, serum, plasma, or a fraction of any of the foregoing;
d. the production of at least one further marker selected from tryptophan-betaine, taurine, valine, biotinidase, and vitamin D binding protein is determined, preferably wherein
i. reduced production of tryptophan-betaine;
ii. reduced production of taurine;
iii. increased production of valine;
iv. increased production of biotinidase; and/or
v. reduced production of vitamin D binding protein
are indicative of the patient's response to said treatment;
e. the production of SERPINA5, tryptophan-betaine, taurine, valine, biotinidase, and vitamin D binding protein is determined, and
i. increased production of SERPINA5;
ii. reduced production of tryptophan-betaine;
iii. reduced production of taurine;
iv. increased production of valine;
v. increased production of biotinidase; and
vi. reduced production of vitamin D binding protein
are indicative of the patient's response to said treatment;
f. the expression is determined by measuring protein production, RNA production, or metabolite production, preferably quantitatively or semi-quantitatively, more preferably by immunoassays, targeted or untargeted assays based on mass spectrometry hyphenated with chromatography;
g. the patient receives a standard treatment of post-viral fatigue syndrome, chronic fatigue syndrome, chronic post-COVID-19 syndrome, or long-COVID;
h. the patient is monitored by determining and comparing the production of the marker(s) at different time points, preferably wherein the blood samples are drawn at least twice, at two or more different time points.

14. A method for identifying a patient eligible for treatment of post-viral fatigue syndrome by determining the production of SERPINA5 in a blood sample of said patient, wherein a reduced production of SERPINA5 is identifying said patient being eligible for treatment.

15. The method of claim 14, which is **characterized by** one or more of the following features:
a. the post-viral fatigue syndrome is chronic post-COVID-19 syndrome or long-COVID;
b. the reduced production of SERPINA5 is indicative of post-viral fatigue syndrome if the production of SERPINA5 is reduced by 30 % compared to a predetermined reference value;
c. the blood sample is whole blood, serum, plasma, or a fraction of any of the foregoing;
d. the production of at least one further marker selected from tryptophan-betaine, taurine, valine, biotinidase, and vitamin D binding protein is determined, preferably wherein
i. increased production of tryptophan-betaine;
ii. increased production of taurine;
iii. reduced production of valine;
iv. reduced production of biotinidase; and/or
v. increased production of vitamin D binding protein
are identifying said patient being eligible for treatment;
e. the production of SERPINA5, tryptophan-betaine, taurine, valine, biotinidase, and vitamin D binding protein is determined, and
i. reduced production of SERPINA5;
ii. increased production of tryptophan-betaine;
iii. increased production of taurine;
iv. reduced production of valine;
v. reduced production of biotinidase; and
vi. increased production of vitamin D binding protein
are identifying said patient being eligible for treatment;
f. the production is determined by measuring protein production, RNA production, or metabolite production, preferably quantitatively or semi-quantitatively, more preferably by immunoassays, targeted or untargeted assays based on mass spectrometry hyphenated with chromatography;
g. the patient receives a standard treatment of post-viral fatigue syndrome, chronic fatigue syndrome, chronic post-COVID-19 syndrome, or long-COVID;
h. the patient is monitored by determining and comparing the production of the marker(s) at different time points, preferably wherein the blood samples are drawn at least twice, at two or more different time points.
